# EUROPEAN PATENT SPECIFICATION

(11) **EP 2 465 944 B1**
(45) Date of publication and mention of the grant of the patent: **17.08.2016**
(21) Application number: 10075758.2
(22) Date of filing: 16.12.2010
(51) Int. Cl.: C12Q 1/68, A61K 38/20

(54) **Method for identifying functional cytokine memory via analysis of DNA methylation**
Verfahren zur Identifizierung eines funktionellen Zytokingedächtnisses mittels einer Analyse der DNA-Methylierung
Procédé d'identification de mémoire de cytokine fonctionnelle via une analyse de méthylation ADN

(43) Date of publication of application: 20.06.2012
(73) Proprietor: Deutsches Rheuma-Forschungszentrum Berlin, 10117 Berlin (DE)
(72) Inventor: Dong, Jun, 13127 Berlin (DE); Thiel, Andreas, 10965 Berlin (DE)
(74) Representative: Lange, Sven

(56) References cited:
- Ann J Melvin ET AL: "Hypomethylation of the interferon-gamma gene correlates with its expression in primary T-lineage cells", Eur J Immunol, 17 November 2005 (2005-11-17), pages 426-430, XP55004929, DOI: 10.1002/eji.1830250218 Retrieved from the Internet: URL:http://onlinelibrary.wiley.com/store/1 0.1002/eji.1830250218/asset/1830250218_ftp .pdf?v=1&t=grezrdq8&s=31d32f3edecc40557985 d38c0d350b234fca179d [retrieved on 2011-08-16]
- FITZPATRICK ET AL: "Distinct methylation of the interferon gamma (IFN-gamma) and interleukin 3 (IL-3) genes in newly activated primary CD8+ T lymphocytes: regional IFN-gamma promoter demethylation and mRNA expression are heritable in CD44(high)CD8+ T cells.", JOURNAL OF EXPERIMENTAL MEDICINE, vol. 188, no. 1, 1 July 1998 (1998-07-01), pages 103-117, XP55004927, ISSN: 0022-1007
- JOHN NORTHROP ET AL: "Epigenetic remodeling of the IL-2 and IFN-gamma loci in memory CD8 T cells is influenced by CD4 T cells.", THE JOURNAL OF IMMUNOLOGY, vol. 177, no. 2, 1 July 2006 (2006-07-01), pages 1062-1069, XP55004728, ISSN: 0022-1767
- AKIKO MURAYAMA ET AL: "A specific CpG site demethylation in the human interleukin 2 gene promoter is an epigenetic memory", THE EMBO JOURNAL, vol. 25, no. 5, 8 March 2006 (2006-03-08), pages 1081-1092, XP55004730, ISSN: 0261-4189, DOI: 10.1038/sj.emboj.7601012
- DENIS BRUNIQUEL ET AL: "Selective, stable demethylation of the interleukin-2 gene enhances transcription by an active process", NATURE IMMUNOLOGY, vol. 4, no. 3, 1 March 2003 (2003-03-01), pages 235-240, XP55004930, ISSN: 1529-2908, DOI: 10.1038/ni887
- L.-O. TYKOCINSKI ET AL: "A Critical Control Element for Interleukin-4 Memory Expression in T Helper Lymphocytes", JOURNAL OF BIOLOGICAL CHEMISTRY, vol. 280, no. 31, 1 August 2005 (2005-08-01), pages 28177-28185, XP55004733, ISSN: 0021-9258, DOI: 10.1074/jbc.M502038200
- DONG U. LEE ET AL: "Th2 Lineage Commitment and Efficient IL-4 Production Involves Extended Demethylation of the IL-4 Gene", IMMUNITY, vol. 16, no. 5, 1 May 2002 (2002-05-01), pages 649-660, XP55004910, ISSN: 1074-7613, DOI: 10.1016/S1074-7613(02)00314-X
- DONG J. ET AL: 'IL-10 is excluded from the functional cytokine memory of human CD4 memory T lymphocytes' THE JOURNAL OF IMMUNOLOGY vol. 179, no. 4, 01 January 2007, pages 2389 - 2396, XP055066628 ISSN: 0022-1767
- ROBERT A SEDER: 'High-Dose 11-2 and IL-15 Enhance the In Vitro Priming of Naive CD4+ T Cells for IFN-y but Have Differential Effects on Priming for IL-4' THE AMERICAN ASSOCIATION OF IMMUNOLOGISTS vol. 156, no. 7, 01 April 1996, pages 2413 - 2422, XP055170954

## Description

The invention relates to a method for establishing functional cytokine memory for IFN-gamma in a cell and/or cell population via demethylation of one or more regulatory nucleic acid sequences associated with the cytokine-encoding nucleic acid sequence. The invention further relates to a method for identifying functional cytokine memory forIFN-gamma in a cell and/or cell population via analysis of DNA methylation of one or more regulatory DNA sequences associated with a nucleic acid sequence encoding the cytokine. The invention further relates to a memory T cell population generated by the method of the present invention for use in cell therapy, preferably in the treatment of cancer, autoimmune diseases or infectious diseases. Another aspect of the invention relates to a method of disease diagnosis and/or monitoring disease progression, preferably before, during and/or after medical treatment, comprising analysis of memory T cells for identifying functional cytokine memory for IFN-gamma whereby the proportion of memory T cells exhibiting functional cytokine memory for IFN-gamma provides a quantitative or semi-quantitative assessment of disease presence and/or progression.

### Background of the Invention

A unique feature of adaptive immunity is the generation of effector and memory T cells after activation, which are capable of controlling pathogens and mediate more effective protection during re-challenges, respectively¹. Faced with intracellular pathogens, naive CD4⁺ T cells adopt Th1 cell fate to differentiate into effector and memory Th1 cells characterised by producing signature cytokine IFN-gamma. These cells play a key role in mediating intracellular killing and also exert their pro-inflammatory effects in organ-specific autoimmune diseases². During primary activation, expression of the *Ifng*/*IFNG* gene by naïve CD4⁺ T cells is initiated by T-cell receptor (TCR) signalling and instructive IL-12 signalling via lineage-specific transcription factors such as T-bet and IL-12-responsive transcription factor STAT4, respectively, and general transcription factors binding to yet low accessible regulatory regions in the *Ifng*/*IFNG* gene³⁻⁵. During secondary activation, however, the rapid *Ifng*/*IFNG* re-expression by memory Th1 cells is triggered by TCR signalling in the absence of the original instructive signals.

Functional cytokine memory for IFN-gamma is achieved by a network of transcription factors engaging the "poised" epigenetically imprinted regulatory regions in the *Ifng*/*IFNG* gene established from previous activations⁶.

The accessibility for transcription factors to their targets is regulated by the epigenetic modifications, including DNA methylation and covalent histone tail modifications^{7, 8}. In particular, DNA methylation is essential because it is potentially reversible and to date the only proven heritable modification of chromatin⁹. DNA methylation of few individual CpGs in the promoter of the *IFNG*/*Ifng* gene has been shown to be inversely correlated with its expression ¹⁰⁻¹³. It has also been shown that treatment with DNA methylation inhibitors augment IFN-gamma production, even in purified cells that previously did not produce the cytokine¹³⁻¹⁵. In addition, *de novo* methylation of the *IFNG* promoter correlate with reduced IFN-gamma production in T cells infected with human immunodeficiency virus type I¹⁶. Together, these studies have demonstrated an essential role for DNA methylation in determining the capacity of T cells to express *Ifng*/*IFNG.* However, how DNA methylation is regulated and "fixed" during differentiation of memory Th1 cells from naive T-cell precursors remains poorly understood.

Two distinct mechanisms of DNA demethylation have been described to date. Passive demethylation occurs during DNA replication because of a failure to methylate CpGs in the newly synthesized strand if levels of DNA methyltransferase I (Dnmtl) are insufficient¹⁷. Active demethylation, on the other hand, takes place in the absence of DNA replication and cell division^{18,19}. Both passive and active mechanisms have been observed during genome-wide demethylation after fertilization in mouse²⁰. Active demethylation has also been demonstrated in the promoter of the *II2*/*IL2* gene in regulating its expression^{21, 22}.

Analyses of regulatory elements in the *Ifng*/*IFNG* gene have yielded considerable insight into control of Th1 selective expression. For example, the conserved non-coding sequence (CNS)-1 located 5 kb upstream of the *Ifng* gene and 4.2 kb of the human gene has been identified by sequence homology analysis between mouse and man. CNS-1 displays Th1-specific DNase I hypersensitivity and enhancer activity. Transcription factors NFAT and T-bet bind to this region in stimulated Th1 cell lines and augment enhancer activity²³. Moreover, in characterizing the proximal *IFNG* promoter, a minimal stretch of 500 bp has been demonstrated to offer Th1/Tc1 selective expression^{24, 25}. Therefore, it remains unknown whether demethylation changes in these Th1-specific regulatory elements influence Th1 lineage commitment particularly during early Th1 cell differentiation.

T cell therapy represents a promising approach in the treatment of various diseases. One example is adoptive T cell therapy. In contrast to vaccination strategies, adoptive T cell therapy strategies can overcome the *in vivo* constraints that influence the magnitude and avidity of a targeted response. T cells of a given specificity, function, and avidity can be selected *in vitro* and then expanded to achieve *in vivo* frequencies in the blood that are higher than generally attained by current immunization regimens.

One of the major limitations in T cell therapy is the generation, identification and/or selection of effector memory T cells, such as Th1 and Tc1 cells, for treatment. IFN-gamma expression is commonly used for identifying effector memory T cells that are suitable for use in therapy. However, not all IFN-gamma-expressing T cells are in fact memory cells. A population of T cells expressing IFN-gamma comprises of T cells in which IFN-gamma is transiently expressed, but without established cytokine memory, and cells that do exhibit stable cytokine memory. Those cells, which are not memory cells, do not lead to effective in vivo responses when used in various therapeutic applications. T cells that exhibit functional cytokine memory react much faster and more efficiently upon challenge with antigen compared to T cells without cytokine memory.

Known in the art are methods of providing memory T cells, whereby peripheral blood mononuclear cells (PBMCs) are isolated, stimulated with antigen, whereby functional tests need be carried out, essentially to screen cells for cytokine memory before T cell treatment.

Functional tests are typically used in the art to assess whether the T cells exhibit functional cytokine memory before application to a patient. Currently, functional tests for identifying memory T cells in *in vitro* generated antigen-specific T cell lines depend on re-activation of cells by specific antigen in the presence of antigen presenting cells (APC). These tests include measurement of proliferative responses of activated T cells, measurement of cytotoxicity of CD8⁺ T cells, and measurement of intracellular cytokine production such as IFN-gamma. The approaches applied suffer from a number of drawbacks which lead to their practical limitations. For example, the aforementioned tests require freshly prepared APCs usually from the same donor. Both cyropreserved APCs and T cell lines will compromise the outcomes of these functional tests. Therefore, current functional tests are inefficient and complicated, and represent a significant disadvantage of the common approaches preparing T cells for cell therapy.

The present invention relates to DNA methylation changes in cytokine genes, such as in the *IFNG* gene, by which naïve human CD4⁺ T cells differentiate into effector Th1 cells, and thus memorize their function. During Th1 differentiation, selective CpG demethylation occurs at the promoter and CNS-1 as early as 16 hr, independent of cell proliferation and DNA synthesis. Day 2 IFN-gamma-producing cells acquired essential selective demethylation and developed into memory cells displaying complete demethylation of these specific CpGs only when exposed to IL-12. Selective demethylation of the *IFNG* gene potentiates heritable and stable functional IFN-gamma memory during the transition from effector to memory Th1 cells.

### Summary of the Invention

The problem to be solved by the present invention is to overcome the deficiencies known in the prior art. The task of the present invention is the provision of a method for generating and/or identifying functional cytokine memory.

The present invention therefore relates to an in vitro method for establishing functional cytokine memory for IFN-gamma in a cell and/or cell population via demethylation of one or more regulatory nucleic acid sequences associated with the cytokine-encoding nucleic acid sequence, comprising the following steps:
- provision of a cell population comprising CD4+ T-cells,
- subsequent activation of the cell population,
- isolation of an IFN-gamma expressing cell population from the activated cells, and
- application of IL-12 to the isolated IFN-gamma expressing cell population to induce DNA demethylation of one or more regulatory nucleic acid sequences associated with the IFN-gamma-encoding sequence.

The method is characterised in that IL-12 is applied to the cells and/or cell population to induce DNA demethylation.

In another embodiment of the method according to the present invention the IL-12 is applied to the cells and/or cell population between 24 and 72 hours, preferably 48 hours, after initial activation and/or isolation of cytokine-expressing cells.

In another embodiment of the method according to the present invention the IL-12 is applied to the cells and/or cell population continually for 2 to 4 weeks, preferably 3 weeks, after initial activation and/or isolation of non-cytokine-expressing cells.

In another embodiment of the method according to the present invention the method comprises the following steps:
- a T-cell containing cell population, such as PBMC, is isolated,
- the naïve T-cell cell population is subsequently activated, preferably via treatment with antigen and/or irradiated APC,
- cytokine-expressing cells are isolated, preferably via FACS and/or MACS, and
- IL-12 is applied to the isolated cytokine-expressing cells, preferably between 24 and 72 hours, preferably 48 hours, after initial activation and/or isolation of cytokine-expressing cells.

In another embodiment of the present invention the method is characterised in that the cell and/or cell population comprises of CD4+ T cells, preferably helper T cells (Th1), Th1 cells that express IFN-gamma, Th2 cells, Th17 cells, Th9 cells and/or IL-22-producing Th-cell and NK-cell subsets.

Functional memory of signature cytokines from various cell types represent embodiments of the present invention, such as IL-4 memory in Th2 cells, IL-17A and IL-17F memory in Th17 cells, IL-9 memory in Th9 cells, and IL-22 memory in IL-22-producing Th-cell and NK-cell subsets.

In another embodiment of the present invention the method is characterised in that the regulatory DNA sequences are promoter sequences, enhancer sequences, operator sequences or any other regulatory DNA element associated with and/or required for transcription of said cytokine encoding nucleic acid.

In another embodiment of the present invention the method is characterised in that the regulatory DNA sequences are the IFNG promoter, IFNG coding region and/or the IFNG-CNS-1 sequence, preferably according to SEQ ID NO 1, SEQ ID NO 2, SEQ ID NO 3 and/or SEQ ID NO 4.

In another embodiment of the present invention the method is characterised in that demethylation of the regulatory DNA sequences occurs at CpG sequences, preferably CpG1: -4397; CpG2: - 4375; CpG3: -4358; CpG4: -4323; CpG5: -4291; CpG6: -4276; CpG7: -4227 of SEQ ID NO 1, and/or CpG1: -495; CpG2: -372; CpG3: -295; CpG4: -186 of SEQ ID NO 2, and/or CpG1: -186; CpG2: -53; CpG3: +122; CpG4: +128; CpG5: +171 of SEQ ID NO 3, and/or CpG1: +122; CpG2: +128; CpG3: +171; CpG4: +296 of SEQ ID NO 4.

A further aspect of the invention relates to a memory T cell population that exhibits functional cytokine memory for IFN-gamma, produced by the method as described herein.

A further embodiment relates to the memory T cells produced by the method as described herein for use as a medicament.

A further embodiment relates to the memory T cells produced by the method as described herein for use in cell therapy, preferably in the treatment of cancer, autoimmune diseases and/or infectious diseases.

A further aspect of the present invention relates to a method for identifying functional cytokine memory for IFN-gamma in a cell and/or cell population via analysis of DNA methylation of one or more regulatory DNA sequences associated with a nucleic acid sequence encoding the cytokine.

In a preferred embodiment the method for identifying functional cytokine memory for IFN-gamma relates to analyzing DNA methylation of:
- promoter sequences, enhancer sequences, operator sequences or any other regulatory DNA element associated with and/or required for transcription of said cytokine encoding nucleic acid,
- the IFNG promoter, IFNG coding region and/or the IFNG-CNS-1 sequence, preferably according to SEQ ID NO 1, SEQ ID NO 2, SEQ ID NO 3 and/or SEQ ID NO 4, and/or
- CpG sequences, preferably CpG1: -4397; CpG2: -4375; CpG3: -4358; CpG4: -4323; CpG5: - 4291; CpG6: -4276; CpG7: -4227 of SEQ ID NO 1, and/or CpG1: -495; CpG2: -372; CpG3: -295; CpG4: -186 of SEQ ID NO 2, and/or CpG1: -186; CpG2: -53; CpG3: +122; CpG4: +128; CpG5: +171 of SEQ ID NO 3, and/or CpG1: +122; CpG2: +128; CpG3: +171; CpG4: +296 of SEQ ID NO 4.

In a preferred embodiment the method for identifying functional cytokine memory is characterised in that low DNA methylation levels, in comparison to control cells, whereby the control cell is a naïve T cell, a CD4+ T cell and/or a CD8+ T cell that does not express the cytokine, represent the presence of cytokine memory.

In a preferred embodiment the method for identifying functional cytokine memory is characterised in that the analysis of DNA methylation is carried out via bisulfite cloning and sequencing of genomic DNA, direct bisulfite sequencing, bisulfite-specific PCR sequencing, pyrosequencing, methylation-sensitive PCR, using preferably quantitative or real-time PCR, HPLC, mass spectrometry, microarray analysis, methylated DNA immunoprecipitation, methylated CpG amplification coupled to microarrays, or any other method for analyzing DNA methylation.

A further aspect of the invention relates to use of the IFN-gamma promoter and the CNS-1 regulatory sequences, preferably according to SEQ ID NO 1, SEQ ID NO 2, SEQ ID NO 3 and/or SEQ ID NO 4, for the generation and/or identification of functional cytokine memory in T cells, preferably Th1 cells, CD4+ T cells, helper T cells (Th1), Th1 cells that express IFN-gamma, Th2 cells, Th17 cells, Th9 cells and/or IL-22-producing Th-cell and NK-cell subsets.

Another aspect of the invention relates to the use of regulatory sequences of the human *IFNG* gene including the promoter and the conserved non-coding sequence (CNS)-1 for the generation and/or identification of functional cytokine memory and/or for the identification of memory T cells, preferably Th1 cells, as well as effector and central memory CD8+ T-cell subsets. The invention also relates to the use of *IFNG* promoter for the identification of differential capacity of the IFN-gamma production in NK cells, preferably CD56bright and CD56dim NK cell subets.

A further aspect of the invention relates to a method of disease diagnosis and/or monitoring disease progression, preferably before, during and/or after a medical treatment, comprising
- providing a population of memory T cells, preferably CD4+ and/or CD45 RO cells from PBMC, followed by
- execution of the method for identifying functional cytokine memory for IFN-gamma according to the present invention, whereby
- the proportion of memory T cells exhibiting functional cytokine memory for for IFN-gamma provides a quantitative or semi-quantitative assessment of disease presence and/or progression.

In a preferred embodiment the method of disease diagnosis and/or monitoring disease progression is characterised in that the disease is Rheumatoid arthritis, and that increased levels of DNA methylation of regulatory DNA elements, preferably those according to the promoter and CNS-1 sequences of IFNG, in CD4+ memory T cells, in comparison to healthy subjects, indicates disease.

The present invention therefore provides a molecular analytical assay for functional cellular properties. The invention relates to the generation and/or molecular determination of cells that exhibit a particular function, namely heritable and rapid cytokine re-expression (functional cytokine memory). This represents a significant improvement over common approaches known in the art for generation and/or identification of memory T cells that are appropriate for cell therapy applications. Where functional tests were previously applied, the present invention provides a fast and efficient molecular diagnostic assay for functional cytokine memory.

Functional cytokine memory, according to the meaning of the present invention, is the heritable and stable expression of cytokine genes. Functional cytokine memory also relates to rapid re-expression of cytokine upon re-experience with antigen. Functional cytokine memory is evident when original instructive signals are no longer necessary for the induction of cytokine re-expression. Rather, the capacity of cytokine re-expression is maintained after activation, preferably in a constitutive manner. Alternatively, upon stimulation, such as re-experience with a known antigen, the particular cytokine is induced (expressed) without requiring the original instructive signalling involved in inducing cytokine expression upon the cell's initial interaction with antigen. Through this mechanism the cytokine response, namely cytokine expression, is either maintained in a cell or is faster when a cell re-encounters a known antigen. Functional cytokine memory does not relate to transient cytokine expression, but rather the maintained expression and/or ability to rapidly re-express cytokine after antigen stimulation.

In one embodiment of the invention, naïve T cells are isolated from a patient, preferably via markers CD4+, CD45RA+ and/or CD31+. T cells are subsequently activated, preferably via stimulation with antigen under Th1 polarizing conditions. Alternatively, peripheral blood mononuclear cells (PBMCs) are extracted from a patient and stimulated with antigen. Cells expressing IFN-gamma are subsequently selected, preferably using MACS and/or FACS. These cells represent activated effector T cells. IFN-gamma-expressing cells can then be separated and cultured, either in heterogeneous or in clonal fashion.

In one embodiment of the invention isolated IFN-gamma-expressing cells are treated with IL-12, which induces DNA demethylation at one or more regulatory DNA elements of any given cytokine gene, preferably *IFNG.* This treatment occurs preferably at 2 days after initial isolation of IFN-gamma-expressing cells.

Treated cells can be subsequently tested for DNA methylation at one or more regulatory DNA elements of any given cytokine gene, preferably *IFNG,* using the method of the present invention. Cells which exhibit DNA hypomethylation at one or more regulatory DNA elements of said cytokine gene are intended to be used in T cell therapy as memory T cells.

In another embodiment of the present invention, a fraction of cells from in vitro generated Antigen-specific T cell lines/cultures will be either frozen at -80°C prior to analysis or directly subjected to DNA methylation analysis to qualify memory status of T cells. This is done by genomic DNA extraction followed by bisulfite conversion using commercial available kits. Subsequently, a small amount of bisulfite-converted DNA will be used as template for bisulfite-specific PCR using specifically designed primer pairs (Table 1) that flank the regulatory DNA elements of said cytokine gene. These PCR products will then be sequenced either by pyrosequencing technology using specifically designed sequencing primers (Table 1) or by cloning sequencing. When the percentage of DNA demethylation in the tested samples at selective CpG sites of one or more DNA regulatory elements of said cytokine gene attains specific threshold levels, the T cells will be considered as memory T cells. When DNA demethylation reaches more than 50%, preferably 60%, 70%, 75%, 80%, 85%, 90%, or 95%, in comparison to control cells, the T cells are deemed to exhibit functional cytokine memory. Conversely, this measure can be described as, that when cells exhibit levels of DNA methylation below 50%, preferably 45%, 40%, 35%, 30%, 25%, 20%, 15%, 10%, or 5%, in comparison to control cells, the T cells are deemed to exhibit functional cytokine memory. The percentage of DNA demethylation in the tested samples at selective CpG sites of one or more DNA regulatory elements of said cytokine gene, in comparison with control cells, represents the frequency of memory T cells.

Alternatively, real-time PCR will be performed with specifically designed methylation or non-methylation-specific forward and reverse primers and methylation sensitive hybridization probes for one or more DNA regulatory elements of said cytokine gene, and 30 ng bisulfite-converted DNA or respective amount of plasmid standard. Plasmid standards, generated by inserting bisulfite-converted DNA from naïve cells (methylated) and from ex vivo IFN-gamma+ cells (non-methylated) to commercial available plasmid, will be serially diluted to generate standard liner curves.

This invention has several potential therapeutic applications, for example in situations such as in vaccine design, autoimmune diseases and organ transplantations, where modulating T cell responses is required, one or more of regulatory DNA elements of said cytokine gene could be specifically targeted by methylation or demethylation approaches.

### Detailed Description of the invention

Early Th1 cell differentiation is triggered by active, selective, and dynamic demethylation of CpGs at both the promoter and CNS-1 of the *IFNG* gene locus. The present invention relates to the first demonstration that early epigenetic modification of the *IFNG* gene locus by selective CpG demethylation is not only essential for early Th1 effector function but also indispensible for the establishment of stable functional Th1 cytokine memory. Moreover, stable memory Th1 cells are characterised by complete demethylation of these selective CpGs at the promoter and CNS-1. The invention therefore relates to the epigenetic selective CpG demethylation that governs Th1 commitment.

CpGs at the promoter and CNS-1 are hypermethylated in naïve and IFN-gamma-non-producing cells, which is in agreement with the inverse correlation between DNA methylation and *IFNG* expression. CpGs -186, -53 and +171 at the promoter, that are targets for general transcription factors such as CREB/ATF and AP-1, were about 70% methylated in naïve T cells, consistent with results obtained with murine naïve T cells^{32, 33}, implying a limited chromatin configuration that allows fast yet low mRNA expression in the course of primary activation²⁷.

Our demonstration that demethylation of selective CpGs at the *IFNG* gene locus occurred irrespective of cell proliferation and DNA replication shows that early demethylation of the *IFNG* gene during Th1 cell differentiation is regulated by an active mechanism. Active demethylation has been demonstrated in several systems, such as demethylation of DNA in the male pronucleus of the fertilized egg³⁴, of the brain derived neurotrophic factor promoter in primary neuronal cells in response to depolarization^{35,36}, and of the *II2*/*IL2* promoter in activated naïve CD4⁺ T cells^{21, 22}. Active demethylation has been proposed to be mediated by demethylase methyl CpG-binding domain (MBD) 2b or MBD 4 protein^{37, 38}, by DNA repair-like pathway³⁹ and by activation-induced cytidine deaminase (AID)18, 40.

By analysing a three-day time-course of methylation changes in Th1 primed naive-cells, we observed dynamic demethylation at selective CpGs such as -4227, -186 and -53. Interestingly, two recent studies also revealed dynamics of DNA demethylation involved in transcriptional activation of promoters of genes such as estrogen receptor α (ERα)-responsive gene *pS2*^{41, 42} In respect to cytokine gene regulation, it was shown a direct structure-function relationship between the spatial organization of the chromatin around the murine *Ifng* gene and its transcriptional potential⁴³. Thus, a role for the dynamic demethylation during early Th1 differentiation could be associated with transcriptional activation of the *IFNG* gene that allows for certain flexibility to maintain or switch-off gene expression in response to distinct stimuli, and/or in conjunction with dynamic alterations of chromatin conformation.

Importantly, the impact of selective CpG demethylation at the two Th1-specific regulatory regions in the *IFNG* gene locus on the formation of memory cells is reflected by the d2-IFN-gamma⁺ and - IFN-gamma⁻ cell-associated differential demethylation status and their disparate cell fates. Thus when receiving continuous IL-12 signal, d2-IFN-gamma⁺ cells with initial level of selective CpG demethylation are able to sustain their effector function accompanied by reinforced demethylation of these selective CpGs; while primed d2-IFN-gamma⁻ cells need repetitive IL-12 signals to be induced to reexpress *IFNG.* Notably, the frequency of IFN-gamma-producing cells from IL-12 cultured d2-IFN-gamma⁻ cells remains consistent after further IL-12 treatment but decreases in the absence of IL-12, suggesting that a fraction of positive cells represents a situation akin to d2-IFN-gamma⁺ cells. It is most likely because demethylation pattern of d2-IFN-gamma⁻_IFN-gamma⁺ cells sorted from 1° +rlL12 cultured d2-IFN-gamma⁻ cells resembled those of early d2-IFN-gamma⁺ cells, indicating under certain circumstances activated non-early-effector Th1 cells maybe screwed towards effector or memory-like Th1 cells. In this regard, it has been shown that mouse TCR-Tg IFN-gamma⁻ cells can acquire effector/memory phenotype *in vivo* with a delayed differentiation program⁴⁴.

The examples show that IL-12 signal acts on d2-IFN-gamma⁺ cells exhibiting a relative poised epigenetic modification of the *IFNG* gene to further deconvolute and to imprint the epigenetic state. Such demethylation imprinting of selective CpG sites at the proximal promoter and CNS-1 provides direct accessibility for binding lineage-transcription factors such as T-bet and other general transcription factors - the basis for functional cytokine memory. In addition, repetitive IL-12 signals also lead to the inverse correlation of selective demethylation of the *IFNG* gene and its expression in primed d2-IFN-gamma⁻ cells. Apparently IL-12 is required not only to induce human Th1 cells but also to establish stable Th1 effector programs at multiple time points of Th1 differention. Our findings are in agreement with recent data described for mouse Th1 cells that enhanced IL-12 signaling through T-bet in the late phase of Th1 priming correlates strongly with frequency of IFN-gamma-producing cells in a later recall response²⁶. Furthermore, a role for STAT4 in driving Th1 differentiation has been shown by STAT4-dependent epigenetic changes in histone modifications^{46, 47}.

Selective and dynamic demethylation of the *IFNG* gene locus driven by TCR and Th1 instruction signals, such as IL-12, induce and stabilize heritable and stable *IFNG* gene expression during the transition from effector to memory Th1 cells.

The present invention is therefore particularly relevant for modulating Th1 responses in vaccine design and in autoimmune diseases and organ transplantations.

### Nucleic acid sequences of the present invention

Genomic sequence of the CNS-1 of *IFNG* (7 CpGs / 425 bp, 5'-3', minus strand), SEQ ID NO 1: 
AAGCAGGACCATGAATCTCTGGATTATCAATACACAATCCCATCTGATATAAAATTAAGAATA
TTGTAATCTGATGATTGTACTGTGTAAGTAGCATACCTTCTATATTCTTTGAACCACTCCTCAA
AAATGGCCAGAAGGCACAAAAGAAAAGGGGGATTCAGAAAAATCTTCCTCTAACTTACG1G
GATTTTATGAGTCAAATTGCG2GGGAAAATCTGACTCCG3TGTGGACTTCTATTCTGTATGTCA
TGCTTCTTCCG4TAGGTTTGGCTTGCATGATGTCAGGAGTTCCG5GAAGTCAAAATAGCG6TG
GGGGCATGGATCCAATATTGACTCATATTGAGTGGAAGAAATCTACG7CCAAAGTCTGGATG
AGATAATTTGGTTGTGAGTGCTAGGATGTGACTGCTGATGACAGAGGAAAAGGG
CpG1, -4397;
CpG2, -4375;
CpG3, -4358;
CpG4, -4323;
CpG5, -4291;
CpG6, -4276;
CpG7, -4227.

Genomic sequence of amplicon 1 at the promoter of *IFNG* (4 CpGs / 342 bp, 5'-3', minus strand), SEQ ID NO 2: 
CCTCACCAAATTGTTCTTTTAACCG1CATTCTTTCCTTGCTTTCTGGTCATTTGCAAGAAAAAT
TTTAAAAGGCTGCCCCTTTGTAAAGGTTTGAGAGGCCCTAGAATTTCG2TTTTTCACTTGTTC
CCAACCACAAGCAAATGATCAATGTGCTTTGTGAATGAAGAGTCAACATTTTACCAGGGCG3A
AGTGGGGAGGTACAAAAAAATTTCCAGTCCTTGAATGGTGTGAAGTAAAAGTGCCTTCAAAG
AATCCCACCAGAATGGCACAGGTGGGCATAATGGGTCTGTCTCATCG4TCAAAGGACCCAAG
GAGTCTAAAGGAAACTCTAACTACAACACCCA
CpG1, -495;
CpG2, -372;
CpG3, -295;
CpG4, -186.

Genomic sequence of amplicon 2 at the promoter of *IFNG* (5 CpGs / 468 bp, 5'-3', minus strand), SEQ ID NO 3: 
CTTGAATGGTGTGAAGTAAAAGTGCCTTCAAAGAATCCCACCAGAATGGCACAGGTGGGCAT
AATGGGTCTGTCTCATCG¹TCAAAGGACCCAAGGAGTCTAAAGGAAACTCTAACTACAACAC
CCAAATGCCACAAAACCTTAGTTATTAATACAAACTATCATCCCTGCCTATCTGTCACCATCT
 
CATCTTAAAAACTTGTGAAAATACG2TAATCCTCAGGAGACTTCAATTAGGTATAAATACCAG
CAGCCAGAGGAGGTGCAGCACATTGTTCTGATCATCTGAAGATCAGCTATTAGAAGAGAAAG
ATCAGTTAAGTCCTTTGGACCTGATCAGCTTGATACAAGAACTACTGATTTCAACTTCTTTGG
CTTAATTCTCTCG3GAAACG4ATGAAATATACAAGTTATATCTTGGCTTTTCAGCTCTGCATCG5
TTTTGGGTTCTCTTGGCTGTTACTGCCAGGAC
CpG1, -186;
CpG2, -53;
CpG3, +122;
CpG4, +128;
CpG5, +171.

Genomic sequence of amplicon 3 at the promoter of *IFNG* (4 CpGs / 434 bp, 5'-3', minus strand), SEQ ID NO 4: 
CCTTTGGACCTGATCAGCTTGATACAAGAACTACTGATTTCAACTTCTTTGGCTTAATTCTCT
CG1GAAACG2ATGAAATATACAAGTTATATCTTGGCTTTTCAGCTCTGCATCG3TTTTGGGTTC
TCTTGGCTGTTACTGCCAGGACCCATATGTAAAAGAAGCAGAAAACCTTAAGAAATATTTTGT
AAGTATGACTTTTTAATAGTACTTGTTTGTGGTTGAAAATGACTGAATATCG4ACTTGCTGTAG
CATCTCTGATAGGCTGTCATCTCTTGTAGGCAGTCATTTTGAGATTTGGTGTTATTTTGTTAAT
TATTGACTAGATGAGTTCCTTGACTAAATAATCTAGATATTGTTTTAACCTTCTGCTCAGTTTG
TATAGAGACTTAAAAGGGATTTATGAATTTTCCAAAAGATGGGCATAATATGGGTA
CpG1, +122;
CpG2, +128;
CpG3, +171;
CpG4, +296.

### FIGURES

- **Figure 1.**: Inverse correlation of methylation at the *IFNG* gene locus and its expression in CD4⁺ T cells.
- **Figure 2.**: Th1 differentiation is accompanied by demethylation of CNS-1 and the promoter of the *IFNG* gene.
- **Figure 3.**: Active and dynamic demethylation at the *IFNG* gene locus during Th1 differentiation.
- **Figure 4.**: Th1 instruction signals and subsequent exposure to IL-12 are necessary and sufficient for developing long-term IFN-gamma memory.
- **Figure 5.**: Distinct DNA methylation profiles of d2-IFN-gamma⁺ and -IFN-gamma⁻ cells, d2-IFN-gamma⁻_IFN-gamma⁺ and d2-IFN-gamma⁻_IFN-gamma⁻ cells, and memory cells at the *IFNG* locus.
- **Figure 6.**: Methylation status of the *IFNG* gene locus in CD8+ T-cell subsets and NK cell subsets.
- **Figure 7.**: Kinetics of mRNA expression for *IFNG, Tbx21,* and *RUNX3.*
- **Figure 8.**: Differential requirement of IL-12 signals in activated d2-IFN-gamma+ and -IFN-gamma- cells for demethylating CpGs at CNS-1 in the IFNG gene locus.
- **Figure 9.**: Comparison of DNA sequence methylation at *IFNG* promoter and CNS-1 sequences in naïve T cells and memory T cells in healthy and disease subjects (Rheumatoid arthritis).

### Detailed description of the Figures

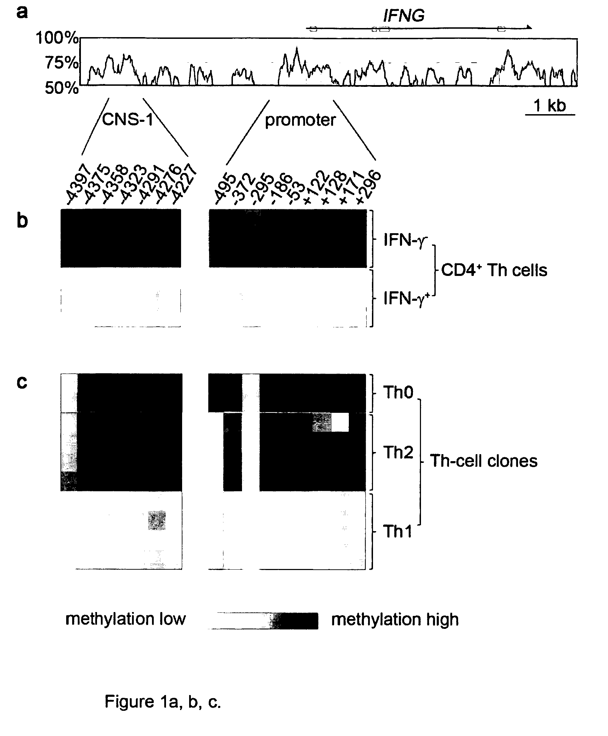

**Figure 1****.** Inverse correlation of methylation at the *IFNG* gene locus and its expression in CD4⁺ T cells. **(a)** Alignment of 10 kb human and mouse *IFNG* gene loci with DNA sequence identity >50% over at least 100 bp are shown in the histogram plot. Arrow, transcription direction; dark blue (area), exons (conserved); light blue (area), UTR (conserved); reddish area, conserved non-coding sequences; individual CpGs at CNS-1 and the promoter are depicted in relation to the transcriptional start site. **(b)** The DNA methylation status of three pooled DNA populations of each IFN-gamma- and IFN-gamma⁺ Th cell subsets and **(c)** two Th0, four Th1 and Th2 clones are shown in rows. DNA methylation levels were determined as described previously¹³.

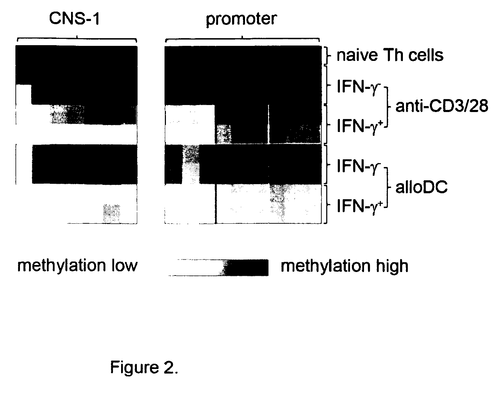

**Figure 2****.** Th1 differentiation is accompanied by demethylation of CNS-1 and the promoter of the *IFNG* gene. The methylation status of pooled (10 donors) naive Th cells, IFN-gamma⁻ and IFN-gamma⁺ Th-cell subsets separated from 1-wk Th1 culture with anti-CD3/28 or alloDC stimulation from two independent experiments are shown in rows. DNA methylation levels were determined as described in figure 1.

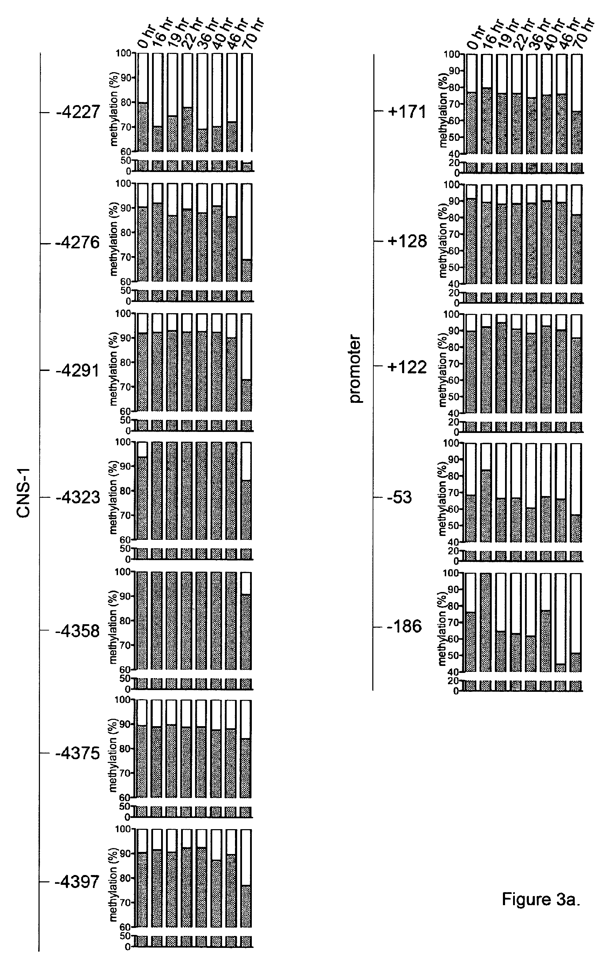

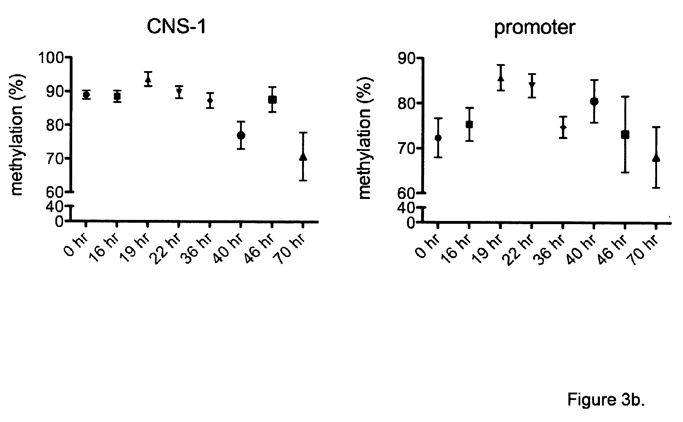

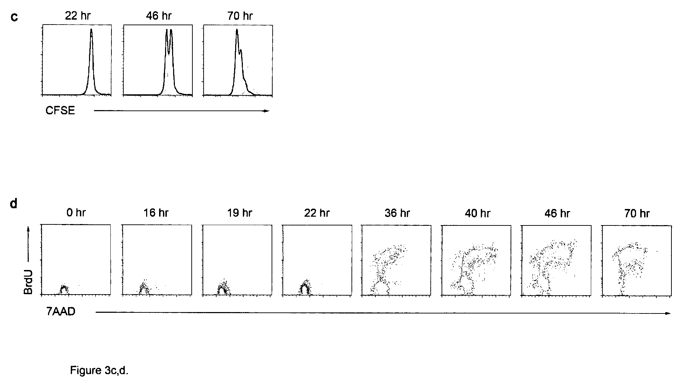

**Figure 3****.** Active and dynamic demethylation at the *IFNG* gene locus during Th1 differentiation. Time-course of methylation status of **(a)** individual CpGs and average level of **(b)** the promoter and CNS-1 during Th1 differentiation with anti-CD3/28 using bisulfite pyrosequecing. Data are from one representative experiment of two independent experiments. **(c)** Time-course of cell proliferation during Th1 differentiation with anti-CD3/28. Overlay of histograms of CFSE-labelled naive cells with (solid line) and without (filled area) priming is shown at indicated time point. The data shown are representative of three independent experiments. **(d)** Time-course analysis of DNA synthesis during Th1 differentiation with cells described in figure 3a.

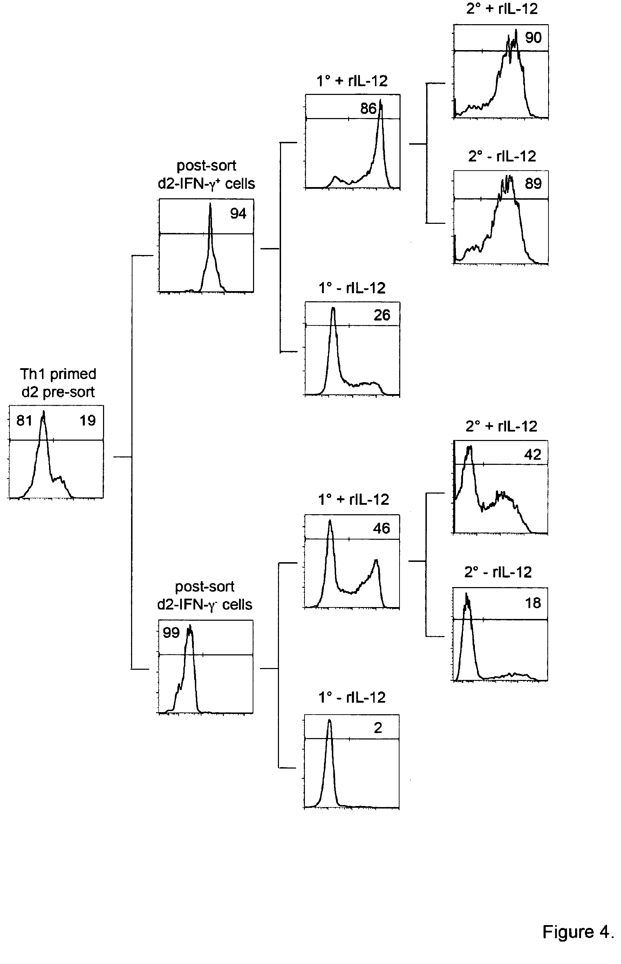

**Figure 4****.** Th1 instruction signals and subsequent exposure to IL-12 are necessary and sufficient for developing long-term IFN-gamma memory. Naïve T cells were primed under a Th1 culture with anti-CD3/28 followed by the IFN-gamma capture assay. Histograms of pre-sort and post-sort are shown. Separated cells were cultured with or without rIL-12 (1° + rIL12; 1° - rIL-12) at d0 and d2. At d7, re-expression of IFN-gamma was assessed by PMA/iono stimulation. Cells that received initial IL-12 treatment were washed and further cultured with or without rIL-12 (2° + rIL12; 2° - rIL-12), and re-expression of cytokine was assessed as described above. Numbers indicate percentage of IFN-gamma-producing cells. The data shown are representative of more than three independent experiments.

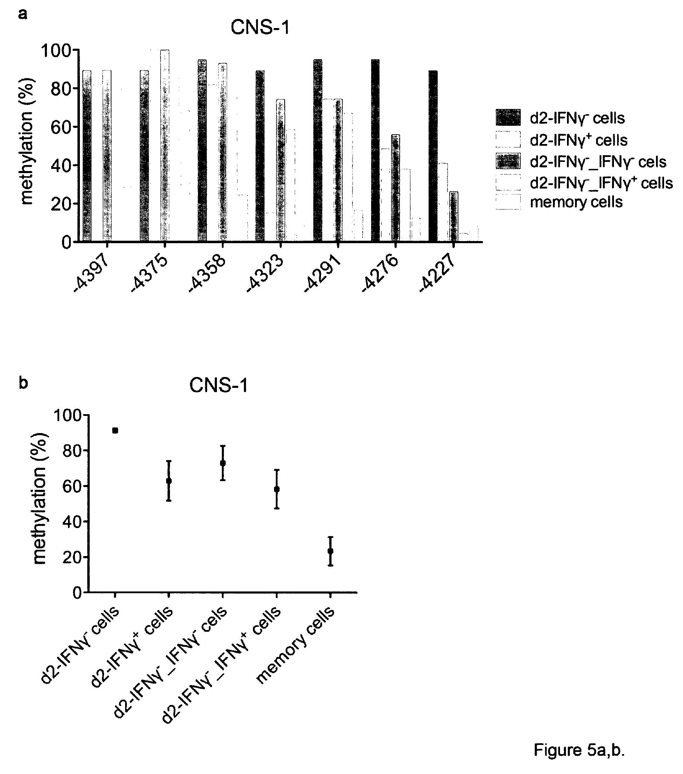

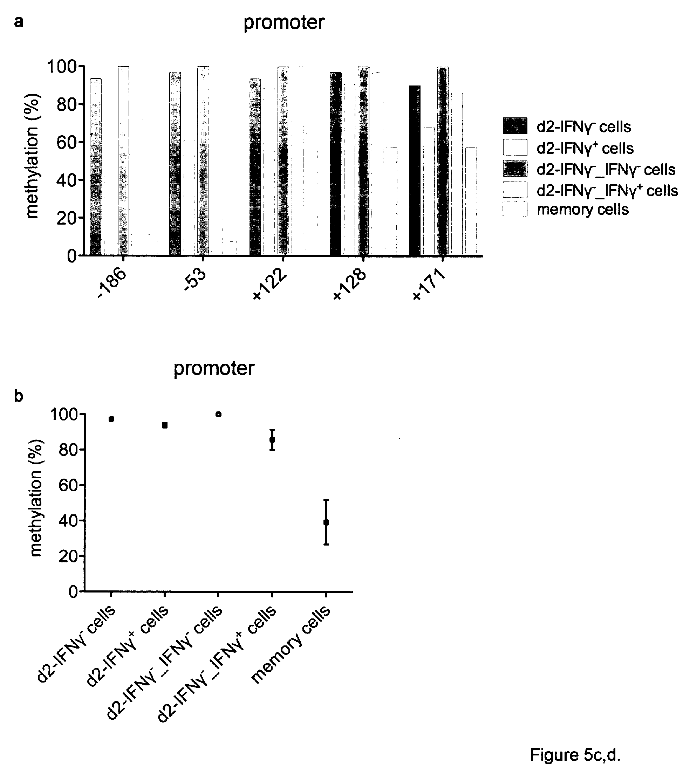

**Figure 5****.** Distinct DNA methylation profiles of d2-IFN-gamma⁺ and -IFN-gamma- cells, d2-IFN-gamma-_IFN-gamma⁺ and d2-IFN-gamma⁻_IFN-gamma⁻ cells, and memory cells at the *IFNG* locus. Methylation of CpG sequences at individual **(a)** and region level **(b)** of CNS-1 and at individual **(c)** and region level **(d)** of the promoter are shown. Dark blue bars, d2-IFN-gamma-cells; light blue bars, d2-IFN-gamma⁺ cells; dark pink bars, d2-IFN-gamma⁻_IFN-gamma⁻ cells; light pink bars, d2-IFN-gamma⁻_IFN-gamma⁺ cells; orange bars, memory cells. Average methylation of the promoter region is depicted as mean with SEM. Data represent two similar independent experiments analyzed by bisulfate cloning sequencing or pyrosequencing (not shown).

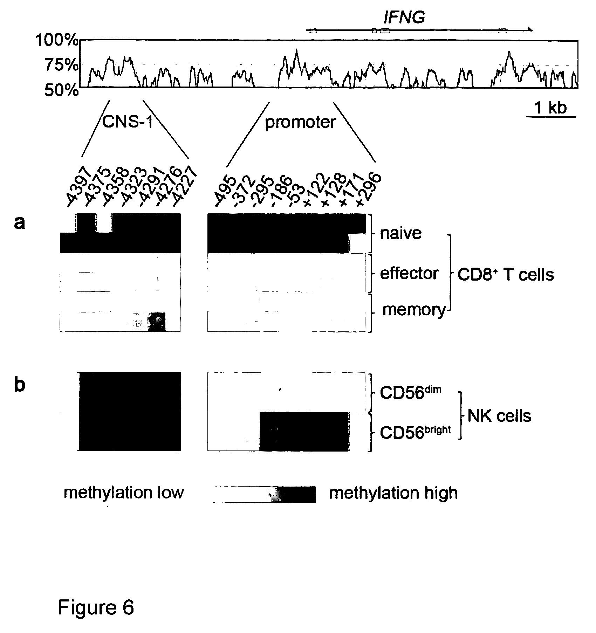

**Figure 6****.** Methylation status of the ***IFNG*** gene locus in CD8+ T-cell subsets and NK cell subsets. CD8+ T cells were purified by MACS positive selection of CD8 microbeads labelled cells followed by FACS sorting of CD3+ (PE; house conjugate), CD4- (PerCP; BD Biosciences) and DAPI-cells. Naive (CD45RA+CD27+), central memory (CD27+CD45RA-), and effector memory (CD45RA+CD27-) CD8+ T-cell subsets were further sorted using the following Abs: anti-CD27 FITC (BD Biosciences), -CD45RA allophycocyanin (Caltag). NK cell subsets, CD56bright and CD56dim cells, were sorted by enrichment of CD56+ cells using NK cell isolation kit (Milenyi Biotec) and in junction with FACS sorting on CD3- (FITC; house conjugate) and CD56+ (PE; Miltenyi) cells. The purity of sorted populations was 95-99% as assessed by FACSCalibur or LSRII using CellQuest (BD Biosciences) or Flowjo (Tree Star) software. DNA methylation levels were determined as described previously. Each rectangle represents one individual CpG and each row represents the methylation status of one donor.

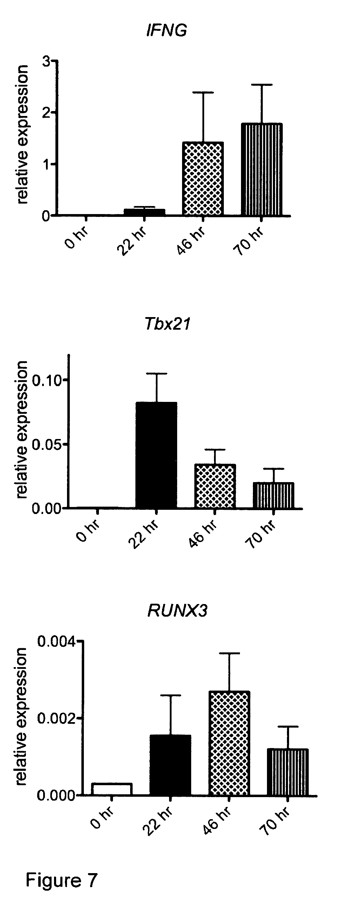

**Figure 7****.** Kinetics of mRNA expression for *IFNG, Tbx21,* and *RUNX3* by naive cells at distinct time-point of Th1 priming.

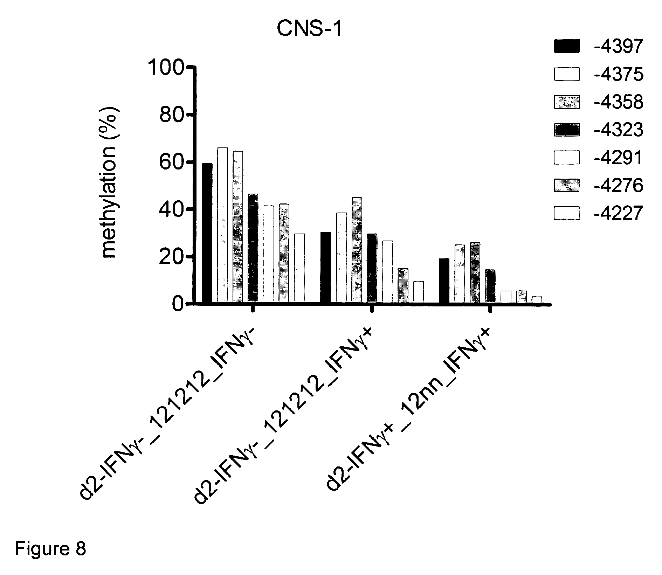

**Figure 8****.** Differential requirement of IL-12 signals in activated d2-IFN-gamma+ and -IFN-gamma-cells for demethylating CpGs at CNS-1 in the IFNG gene locus. d2-IFN-gamma+ and -IFN-gamma- cells were sorted from a 2-day Th1 culture stimulated with anti-CD3/28 in the presence of rIL-12, rIFN-gamma, and anti-IL-4, using cytokine capture assay and FACS. Purified cells were further cultured with rIL-7, and with or without rIL-12 for 3 wk. Cytokine was added twice for every two days per wk. Cells were washed before a next wk of culture. 3 wk-cultured cells were stimulated with PMA/iono for 4 hr (the last 3 hr with Brefeldin A), fixed, pemeabilized and stained for intracellular cytokine IFN-7. Stained cells were FACS sorted into IFN-gamma+ and -IFN-gamma- cells, followed by genomic DNA extraction, bisulfite PCR, and pyrosequencing of indicated CpGs at CNS-1 of the IFNG gene. d2-IFN-gamma-_121212_IFNg-amma-/+, IFN-gamma- and IFN-gamma+ cells derived from d2-IFN-gamma- cells cultured for 3 wk with rIL-12; d2-IFN-gamma+_12nn_IFN-gamma+ cells sorted from d2-IFN-gamma+ cells cultured only 1st wk with rIL-12.

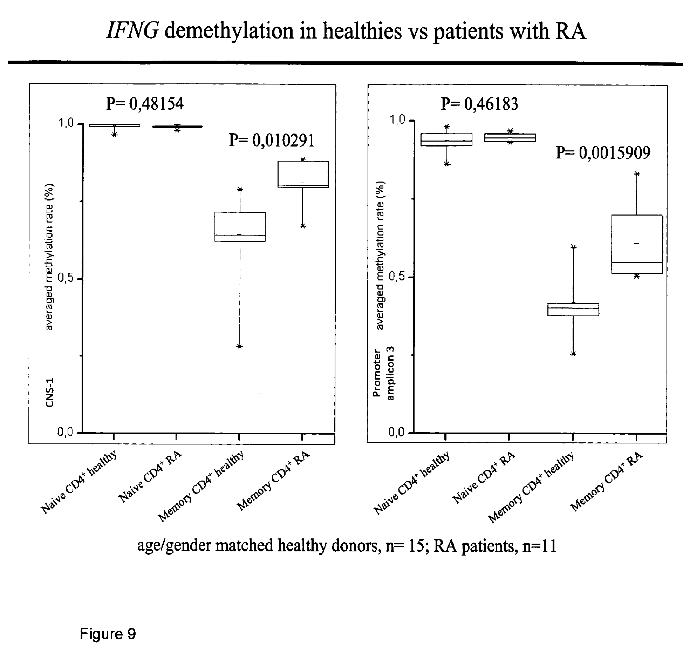

**Figure 9****.** Comparison of DNA sequence methylation at *IFNG* promoter and CNS-1 sequences in naïve T cells and memory T cells in healthy and disease subjects (Rheumatoid arthritis). Ex vivo naïve and memory CD4+ T-cell subsets were isolated from age- and gender-matched healthy and diseased subjects. Methylation status of the CNS-1 and promoter (amplicon 3) were analyzed by bisulfite-specific PCR sequencing as described in figure 1. Percentage of DNA methylation is shown as average of the indicated region among each subject group.

### Methods used in the examples:

### Media and reagents

The media used were RPMI 1640 supplemented with 1% glutamax, 100 U/ml penicillin, 100 µg/ml streptomycin (Invitrogen Life Technologies), and 10% human AB serum (PAA). PMA (5 ng/ml; Sigma-Aldrich) and 1 µg/ml ionomycin (iono; Sigma-Aldrich) were used for stimulation. Brefeldin A (5 µg/ml; Sigma-Aldrich) was used to block cytokine secretion. Recombinant (r) IL-7, rIL15, rIL-12, and rIFN-gamma, each 10 ng/ml (R&D systems), were applied to different cell cultures as indicated in the text.

### Ex vivo cell purification

Buffy coats from healthy adult anonymous donors were obtained in accordance with local ethical committee approval. PBMCs were isolated by density gradient sedimentation using Ficoll-Hypaque (Sigma-Aldrich). Naïve CD4⁺ Th cells defined as CD45RA⁺CD31⁺CD45RO⁻ subpopulation⁴⁸ were sequentially purified: first by isolation of CD4⁺ T cells with CD4 microbeads (Miltenyi Biotec); then by sorting of CD45RA⁺CD31⁺CD45RO⁻CD4⁺ cells via FACSDiva™ (BD Biosciences) according to the CD45RA (allophycocyanin; Caltag) and CD45RO (FITC; BD Biosciences) isoforms and CD31 (PE; BD Biosciences) expression, and dead cell exclusion by DAPI (Caltag) staining. Monocytes were separated from PBMCs by MACS positive selection of CD14⁺ cells using CD 14 microbeads (Miltenyi Biotec). The purity of sorted populations was 95-99%, as assessed by FACSCalibur or LSRII using CellQuest (BD Biosciences) or Flowjo (Tree Star) software.

### Real-time quantitative PCR analysis

Total RNA from ex vivo or primed naïve CD4⁺ Th cells at day 1, 2 and 3 was extracted using RNeasy Mini Kit (Qiagen) and reverse transcribed with TaqMan Reverse transcription reagents (Roche Applied Biosystems) according to the manufacturer's recommendations. cDNA was analyzed for the expression of *IFNG, Tbx21, Runx3* and ubiquitin *UBCH5B* by real-time quantitative PCR analysis as described previously¹³. Primer sequences are available in the Table 1.

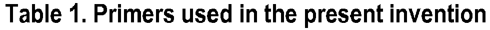

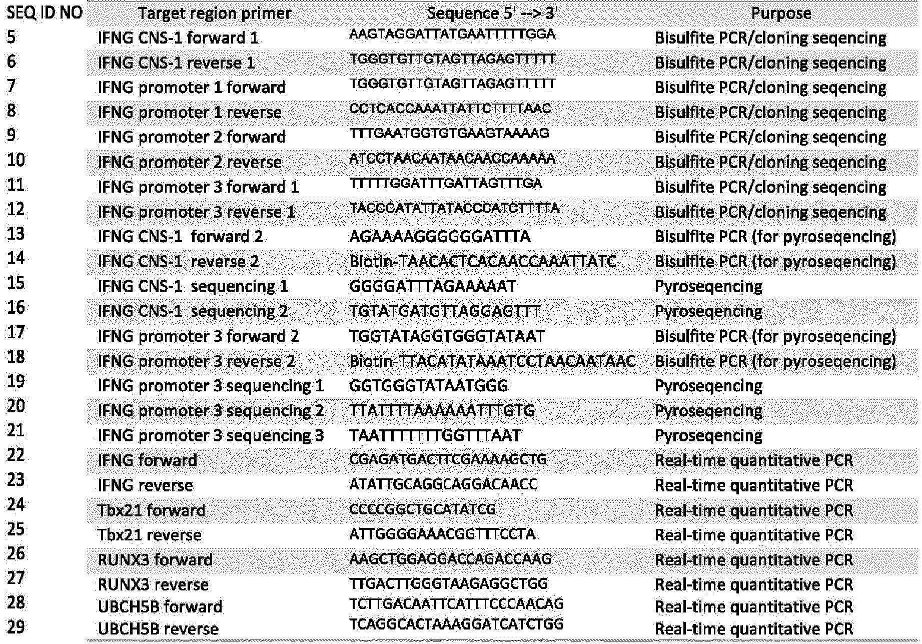

### In vitro generation of DCs

DCs were generated by culturing purified CD14⁺ monocytes (1x10e5 cells/ml), in the presence of 50 ng/ml GM-CSF and 50 ng/ml IL-4 (both R&D Systems) for 5 days and 1 µg/ml LPS (Strathmann Biotech AG) for additional 2 days. CD80, CD83, and CD86 (PE, FITC, and allophycocyanin, respectively; BD Biosciences) expression were used to monitor the maturation status of DCs. More than 80% upregulation of these markers were achieved in all experiments.

### In vitro generation of Th1 cells by alloDCs or TCR stimulation

For Th1 cell differentiation, purified or CFDA,SE-labelled (1 µM) naïve CD4⁺ Th cells (1-2 x 10⁶/ml), where indicated, were primed with CFDA,SE-labelled (5 µM; Molecular Probes) allogenic monocyte-derived DCs (10:1) or plate-bound anti-CD3/CD28 (0,5 µg/ml / 1 µg/ml; BD Biosciences), in the presence of 10 ng/ml rIFN-gamma, rIL-12 (R&D Systems) and anti-IL-4 (10 µg/ml; BD Biosciences) for 2 days followed by expansion on d3 and the collection of resting cells on d7.

### IFN-gamma capture assay

Isolation of Th-cells secreting IFN-gamma was similar as described¹³ with some modifications. Briefly, *in vitro* polarized Th1 cells or highly purified primary CD4⁺ T lymphocytes or human Th1 clone were stimulated with PMA/lono for 4 hr, followed by detection and isolation of IFN-gamma-secreting Th cells using cytometric cytokine secretion assay (Miltenyi Biotec) and a FACSDiva™ (BD Biosciences). In order to prevent non-specific cell labelling and detection, 10 µg/ml and 1 µg/ml anti-IFN-gamma was applied to cell suspension before adding catch reagent and detection Ab, respectively. IFN-gamma-secreting Th cells and their counterpart were also directly isolated after 2- or 3-day priming with plate-bound anti-CD3/CD28 in the presence of rIFN-gamma, rIL-12, and anti-IL-4.

### Intracellular cytokine staining

For IFN-gamma reexpression, naive cells after 2-day priming were removed from stimuli and washed, or sorted to d2-IFN-gamma⁺ and -IFN-gamma⁻ fractions, and subsequently cultured for 1- to 3-wk in either of the following conditions, neutral (rIL-7) or plus rIL-12. After each week of culture fraction of cells were stimulated with PMA/lono for 4 hr (the last 3 hr with Brefeldin A), fixed, permeabilized and stained for intracellular cytokine IFN-gamma (FITC; house conjugate or allophycocyanin; BD Biosceinces). Stained cells were measured by flow cytometry and data analyzed using Flowjo. Alternatively, IFN-gamma⁺ and IFN-gamma⁻ cells were sorted for methylation analysis.

### Bisufite-specific PCR sequencing, cloning sequencing and pyrosequencing

Genomic DNA was extracted using a OIAamp® DNA mini kit (Qiagen), and the bisulfite conversion was done using an EpiTect bisulfite kit (Qiagen) according to the manufacturer's instructions, respectively. Briefly, 100-300 ng genomic DNA was bisulfite converted using a thermal cycler. Cycling conditions were as the follows: 99°C for 5 min, 60°C for 25 min, 99°C for 5 min, 60°C for 85 min, 99°C for 5 min, and 60°C for 175 min. Regions of interest in the IFNG locus was BSP amplified using primers **(Table 1)** designed by using Primer3 software. PCR was performed using the following program: 95°C for 5 min, 95°C for 1 min, 55°C for 45 s, 72°C for 1 min, for 40 cycles, and final extension at 72°C for 10 min. The PCR product was then cloned using the TOPO TA cloning kit (Invitrogen) and sequenced via vector-based primers on both orientations (Eurofins MWG Operon or GATC biotech, Germany). Alternatively, BSP product was directly sequenced using the PCR primers and methylation levels were calculated as previously described¹³. In addition, bisulfite pyrosequencing approach (Varionostic GmbH, Germany) was applied as indicated in the text. Primer sequences for bisulfite PCR and sequencing are listed in Table 1.

### EXAMPLES

The inventors have analysed methylation patterns of the promoter and CNS-1 at the *IFNG* gene locus in IFN-gamma-producing CD4⁺ and CD8⁺ T cells and NK cells. Methylation changes of these regions in CD4⁺ T cells during their differentiation from naive to IFN-gamma-producing cells were also interrogated. These examples show that initiation of selective demethylation of the *IFNG* gene locus is mediated by active demethylation. It is also shown that early-differentiated d2-IFN-gamma⁺ and -IFN-gamma⁻ cells differ in their ability to re-express *IFNG* as a result of demethylation of these selective CpGs at the promoter and CNS-1 of the *IFNG* gene locus. In addition, development of competent chromatin structure for stable functional cytokine memory for IFN-gamma required IL-12 signalling at multiple time points of Th1 differentiation. This invention therefore relates to the epigenetic determination of stable memory Th1 cell differentiation.

### Inverse and heritable correlation of DNA methylation of the IFNG gene locus and its expression

At first we identified regions of interests (ROI) at CNS-1 encompassing 7 CpG sites in the *IFNG* locus 4,2 kb upstream from the transcriptional start site and at the promoter 8 CpG sites by aligning man and mouse genomic DNA sequences using the VISTA tool **(****Fig. 1a****).**

To correlate the levels of DNA methylation with its expression, we isolated *ex vivo* IFN-gamma-secreting and non-secreting Th-cell subsets using the cytokine capture assay and performed bisufite-specific PCR sequencing. Overall, *ex vivo* IFN-gamma⁺ and IFN-gamma⁻ Th cells displayed hypo- and hyper-methylation patterns, respectively **(****Fig. 1 b)****.** Similar to CD4⁺ Th-cell subsets, naïve CD8⁺ cells were hypermethylated and effector and memory CD8⁺ cells hypomethylated **(****Fig.6a****).** In contrast, NK cells displayed different methylation patterns compared with those by CD4⁺ and CD8⁺ T cells **(****Fig. 6b****).** The methylation patterns of CD4⁺ and CD8⁺ T cells and NK cells confirmed that the promoter and CNS-1 offer epigenetic T-cell selective regulation.

We next examined methylation patterns in Ag-specific Th clones. Th1 clones (IFN-gamma, 97%; IL-4, 12%) displayed similar hypomethylation pattern as *ex vivo* IFN-gamma⁺ Th cells, in comparison with Th0 (IFN-gamma, 13%; IL-4, 38%) and Th2 (IFN-gamma, 3%; IL-4, 78%) clones (data not shown, **Fig. 1c**).

Taken together, these results demonstrate that heritable hypomethylation of the *IFNG* gene in T-cell specific regulatory regions of the *IFNG* gene locus correlated with its expression in CD4⁺ T cells.

### Demethylation of the IFNG gene locus during Th1 cell differentiation

The clear correlation between the methylation status of the *IFNG* gene and its expression suggested that there must be a demethylation process involved in regulating the *IFNG* expression during differentiation of naïve IFN-gamma-non-producing Th cells to effector/memory IFN-gamma-producing Th cells. To test this hypothesis, we analyzed methylation changes of the *IFNG* gene promoter and CNS-1 in naïve CD4⁺ T cells and in 1-wk *in vitro* differentiated IFN-gamma⁺ and IFN-gamma⁻ Th-cell subsets. As expected, naïve Th cells showed hypermethylation in both the promoter and CNS-1 regions. IFN-gamma⁺ and IFN-gamma⁻ Th-cell subsets displayed hypo- and hyper-methylation, respectively **(****Fig. 2****).** However, in comparison with *ex vivo* IFN-gamma⁺ cells, d7-IFN-gamma⁺ cells seemed to be less demethylated in these two regulatory regions. In this respect, d7-IFN-gamma⁺ and -IFN-gamma⁻ cells isolated from Th1 cultures with alloDCs showed overall more demethylation than those from Th1 cultures with anti-CD3 and - CD28. These methylation data confirm that differentiation of effector/memory IFN-gamma-producing Th cells from naïve precursors is accompanied by demethylation of the *IFNG* gene.

### Demethylation of the IFNG gene through active and dynamic process

To assess demethylation of the *IFNG* gene triggered by early Th1 cell differentiation, we performed a three-day time-course analysis of methylation changes in naïve Th cells activated with plate-bound anti-CD3 and -CD28 under Th1 condition using bisulfate pyrosequencing technology. In *ex vivo* naïve Th cells (0 hr), CpGs at CNS-1 were methylated between 80%-100% and CpGs at the promoter between 68%-91%. Of note, after 16 hr activation, methylation levels of CpG -4227 at the CNS-1 was reduced by 10% from 80% to 70% methylation. Similarly, after 19 hr activation, 13% reduction in methylation from 76% to 63% was detected for CpG -186 at the promoter. In addition, between time-points 16 hr and 19 hr, 15% decrease in methylation levels for CpG -53 and 35% for CpG -186 at the promoter were also observed. Moreover, progressive demethylations increased over an activation period of 70 hr at individual CpGs **(****Fig. 3a****)** as well as at mean levels of demethylation of the promoter and CNS-1 **(****Fig. 3b****).**

We next assessed a possible relationship between methylation changes and cell divisions. No cell division was detected at 22 hr of a Th1 culture as measured by CFSE dilution on CFSE labelled naive Th cells. At 46 hr one cell division was observed and 70 hr was required for most of the cells to divide one or more times **(****Fig. 3c****).** Activation of cells over time was paralleled with levels of *IFNG* expression and Th1-lineage transcription factor *Tbx21* and *RNX3* expression **(****Fig. 7****),** in agreement with recent results described for mouse Th1 cells²⁶.

To define whether and when the activated cells had synthesized DNA, we matched the methylation time-course analysis with BrdU incorporation assay. No BrdU⁺ cells were detected at time points 16 hr, 19 hr and 22 hr. In contrast, increasing amounts of BrdU⁺ cells were observed between 36 hr and 70 hr **(****Fig. 3d****).**

Together, these data demonstrate that early demethylation of the *IFNG* gene does not require DNA replication. Moreover, the overall demethylation over time appears to be selective and dynamic.

### Development of functional IFN-gamma memory at multiple time points via IL-12 signalling

We next investigated how and when naïve human CD4⁺ T cells develop functional cytokine memory for *IFNG* reexpression after primary activation. While levels of expression of *IFNG* mRNA could be detected as early as 2 hr after activation²⁷, IFN-gamma-producing effector cells emerged at day 1 (< 0,5%) and increased over time (d2, 8-19%; d3, 30-40%) upon Th1 polarization with anti-CD3 and CD28 stimulation (**Fig. 4** and data not shown). To assess whether cells that differ in their initial ability to secret IFN-gamma have differential capacity to become functional IFN-gamma memory cells, we sorted IFN-gamma⁺ and IFN-gamma⁻ cells at d2 of Th1 culture using the cytokine capture assay. Because IL-12, the major instructive signal of Th1 differentiation, is postulated to play a role in sustaining Th1 cell immunity *in vivo* in several experimental models^{3,28-30}, we examined the effect of IL-12 on the fate of d2-IFN-gamma⁺ and d2-IFN-gamma⁻ cells. Separated cells were cultured in the presence or absence of IL-12 for 3 days and restimulated with PMA/iono at day 8. In response to initial IL-12 signal (1° +rIL-12), d2-IFN-gamma⁺ cells were able to re-express *IFNG* by 86%; d2-IFN-gamma⁻ cells were induced to express *IFNG* by 45%. In contrast, in the absence of IL-12 (1°-rIL-12), majority of d2-IFN-gamma⁺ cells lost their ability to reproduce IFN-gamma; d2-IFN-gamma⁻ cells remained negative for IFN-gamma production. Consistent with previous observations^{5, 31}, the responses to IL-12 by d2-IFN-gamma⁺ and -IFN-gamma⁻ cells were mediated via IL-12/STAT4 signalling.

In order to address whether initial exposure to IL-12 was sufficient for d2-IFN-gamma⁺ cells to become stable memory cells and, for d2-IFN-gamma⁻ cells to maintain the induced *IFNG* expression, cells at d8 were further cultured with or without IL-12 and restimulated as described earlier. D2-IFN-gamma⁺ cells that had received initial IL-12 signal were able to re-express *IFNG* by 90% and 89% in response to further with and without IL-12 treatments (2° +rIL-12; 2° -rIL-12), respectively (**Fig. 4**). Notably, IL-12 treated d2-IFN-gamma⁻ cells in response to second round of treatments (2° +rIL-12; 2° -rIL-12) behaved similarly like d2-IFN-gamma⁺ cells in response to first round of treatments (1° +rIL-12, 1° -rIL-12), respectively (**Fig. 4**).

Taken together, these data show that cells which differ in their ability to produce IFN-gamma during primary activation adopt differential fates in response to subsequent IL-12 signal, and that development of stable functional cytokine memory for IFN-gamma requires IL-12 signalling at multiple time points of Th1 differentiation.

### Epigenetic mechanism for differential fates of d2-IFN-gamma⁺ and -IFN-gamma⁻ cells

Both d2-IFN-gamma⁺ and -IFN-gamma- cells responded to 1° IL-12 application, whereas only d2-IFN-gamma⁺ cells promptly adopted stable memory fate led us to dissect the accessibility of Th1-specific regulatory elements of the *IFNG* gene imprinted during early Th1 differentiation. We therefore performed methylation analysis with early differentiated d2-IFN-gamma⁺ and d2-IFN-gamma- cells, 1° IL-12 treated d2-IFN-gamma- cell-induced IFN-gamma⁺ (d2-IFN-gamma⁻_IFN-gamma⁺) and IFN-gamma- cells (d2 -IFN-gamma⁻_IFN-gamma⁻), and 1 IL-12 treated d2-IFN-gamma⁺ cells that developed memory for *IFNG* reexpression (memory cells).

At CNS-1, d2-IFN-gamma- cells were hypermethylated in all CpG sites; d2-IFN-gamma⁺ cells were overall hypomethylated. In particular, in comparison with naive cells, d2-IFN-gamma⁺ cells acquired 79% demethylation at CpG -4323 and, 52%, 40%, 19% and 18% at CpGs -4276, -4227, -4358 and -4291, respectively. Strikingly, in memory cells, demethylation of these CpGs were very much pronounced and almost reached to maximal (76-86%) in comparison with naive cells, when 86% of IFN-gamma⁺ cells in analyzed population **(****Fig. 4****)** were accounted. In addition, considerable demethylation (21 % and 62%) was also detected at other two CpGs (-4397 and - 4375) in memory cells when compared with naive cells. Interestingly, while d2-IFN-gamma⁻_IFN⁻ gamma⁺ cells displayed similar demethylation pattern as d2-IFN-gamma⁻⁺ cells, d2-IFN-gamma⁻-_IFN-gamma⁻ cells hypomethylated similarly as d2-IFN-gamma- cells **(****Fig. 5a****).** Moreover, methylation changes during Th1 differentiation in analysed cell types were also represented by their differential averages of methylations at regional level **(****Fig. 5b****).** Finally, memory cells were able to maintain their demethylation status without further IL-12 signal; while activated d2-IFN-gamma⁻ cells required repetitive IL-12 signals to induce demethylation of these spelective CpGs at CNS-1 **(****Fig. 8****).**

Of notice, imprinting of the promoter between analysed cell types mirrored that of the CNS-1. Especially, in comparison with naive cells, 60% and 61% demethylation by memory cells were observed at CpGs -186 and -53, respectively **(****Fig. 5c****).** Again, demethylation at these two CpGs reached to maximal when 86% of IFN-gamma⁺ cells in analyzed population **(****Fig. 4****)** were accounted. Similar to CNS-1, methylation changes were relatively represented by their differential averages of methylations at regional level **(****Fig. 5d****).**

Together, the examples described herein demonstrate that initial epigenetic modification by selective hypomethylation of CpGs at Th1-specific regulatory regions of the *IFNG* locus is essential for early effector Th1 cells to become memory Th1 cells in response to IL-12. The memory Th1 cells are characterized by stable *IFNG* expression and complete demethylation of these selective CpGs.

### Disease Monitoring

Rheumatoid arthritis (RA) is a chronic, systemic inflammatory autoimmune disease that my affect many tissue and organs, but preferentially attacks the synovial joints. Many lines of evidence suggest the presence of Th1 activity in the joint and reduced Th1 response in the peripheral, thus supporting the hypothesis of selective recruitment of effector/memory Th1 cells at the site of inflammation. To address this hypothesis, we compared the distribution of IFN-gamma-producing cells among naïve and memory CD4+ T-cell subsets between age- and gender-matched healthy and RA subjects. The comparison was performed by analysing average of DNA methylation status of the IFNG promoter and CNS-1 region (Fig. 9). In naïve T cell compartment, there were no differences in average of DNA methylation levels between healthy controls and RA patients. By contrast, significant differences in average of DNA methylation at both the CNS-1 and promoter were detected among memory T-cell compartment between these two subject groups. These differences in DNA methylation correlated with differential frequencies of IFN-gamma-producing cells among memory T cells between two subject groups (data not shown). Our data demonstrate that DNA methylation analysis of *IFNG* as a novel method for analysing distribution and/or re-distribution of effector/memory IFN-gamma-producing cells in human peripheral blood. Thus the usage of such analysis represents a very useful tool for disease monitoring.

### References

1. Ahmed, R. & Gray, D. Immunological memory and protective immunity: understanding their relation. Science 272, 54-60 (1996).
2. Mosmann, T.R., Cherwinski, H., Bond, M.W., Giedlin, M.A. & Coffman, R.L. Two types of murine helper T cell clone. I. Definition according to profiles of lymphokine activities and secreted proteins. J Immunol 136, 2348-2357 (1986).
3. Seder, R.A., Gazzinelli, R., Sher, A. & Paul, W.E. Interleukin 12 acts directly on CD4+ T cells to enhance priming for interferon gamma production and diminishes interleukin 4 inhibition of such priming. Proc Natl Acad Sci U S A 90, 10188-10192 (1993).
4. Szabo, S.J. et al. A novel transcription factor, T-bet, directs Th1 lineage commitment. Cell 100, 655-669 (2000).
5. Thierfelder, W.E. et al. Requirement for Stat4 in interleukin-12-mediated responses of natural killer and T cells. Nature 382, 171-174 (1996).
6. Lohning, M., Richter, A. & Radbruch, A. Cytokine memory of T helper lymphocytes. Adv Immunol 80, 115-181 (2002).
7. Ansel, K.M., Lee, D.U. & Rao, A. An epigenetic view of helper T cell differentiation. Nat Immunol 4, 616-623 (2003).
8. Wilson, C.B., Rowell, E. & Sekimata, M. Epigenetic control of T-helper-cell differentiation. Nat Rev Immunol 9, 91-105 (2009).
9. Bird, A. DNA methylation patterns and epigenetic memory. Genes Dev 16, 6-21 (2002).
10. White, G.P., Watt, P.M., Holt, B.J. & Holt, P.G. Differential patterns of methylation of the IFN-gamma promoter at CpG and non-CpG sites underlie differences in IFN-gamma gene expression between human neonatal and adult CD45RO- T cells. J Immunol 168, 2820-2827 (2002).
11. Fitzpatrick, D.R. et al. Distinct methylation of the interferon gamma (IFN-gamma) and interleukin 3 (IL-3) genes in newly activated primary CD8+ T lymphocytes: regional IFN-gamma promoter demethylation and mRNA expression are heritable in CD44(high)CD8+ T cells. J Exp Med 188, 103-117 (1998).
12. Melvin, A.J., MCGurn, M.E., Bort, S.J., Gibson, C. & Lewis, D.B. Hypomethylation of the interferon-gamma gene correlates with its expression by primary T-lineage cells. Eur J Immunol 25, 426-430 (1995).
13. Dong, J. et al. IL-10 is excluded from the functional cytokine memory of human CD4+ memory T lymphocytes. J Immunol 179, 2389-2396 (2007).
14. Farrar, W.L., Ruscetti, F.W. & Young, H.A. 5-Azacytidine treatment of a murine cytotoxic T cell line alters interferon-gamma gene induction by interleukin 2. J Immunol 135, 1551-1554 (1985).
15. Young, H.A. et al. Differentiation of the T helper phenotypes by analysis of the methylation state of the IFN-gamma gene. J Immunol 153, 3603-3610 (1994).
16. Mikovits, J.A. et al. Infection with human immunodeficiency virus type 1 upregulates DNA methyltransferase, resulting in de novo methylation of the gamma interferon (IFN-gamma) promoter and subsequent downregulation of IFN-gamma production. Mol Cell Biol 18, 5166-5177 (1998).
17. Brandeis, M. et al. Sp1 elements protect a CpG island from de novo methylation. Nature 371, 435-438 (1994).
18. Bhutani, N. et al. Reprogramming towards pluripotency requires AID-dependent DNA demethylation. Nature 463, 1042-1047.
19. Gjerset, R.A. & Martin, D.W., Jr. Presence of a DNA demethylating activity in the nucleus of murine erythroleukemic cells. J Biol Chem 257, 8581-8583 (1982).
20. Oswald, J. et al. Active demethylation of the paternal genome in the mouse zygote. Curr Biol 10, 475-478 (2000).
21. Bruniquel, D. & Schwartz, R.H. Selective, stable demethylation of the interleukin-2 gene enhances transcription by an active process. Nat Immunol 4, 235-240 (2003).
22. Murayama, A. et al. A specific CpG site demethylation in the human interleukin 2 gene promoter is an epigenetic memory. EMB J 25, 1081-1092 (2006).
23. Lee, D.U., Avni, O., Chen, L. & Rao, A. A distal enhancer in the interferon-gamma (IFN-gamma) locus revealed by genome sequence comparison. J Biol Chem 279, 4802-4810 (2004).
24. Penix, L.A. et al. The proximal regulatory element of the interferon-gamma promoter mediates selective expression in T cells. J Biol Chem 271, 31964-31972 (1996).
25. Soutto, M. et al. A minimal IFN-gamma promoter confers Th1 selective expression. J Immunol 169, 4205-4212 (2002).
26. Schulz, E.G., Mariani, L., Radbruch, A. & Hofer, T. Sequential polarization and imprinting of type 1 T helper lymphocytes by interferon-gamma and interleukin-12. Immunity 30, 673-683 (2009).
27. Letimier, F.A., Passini, N., Gasparian, S., Bianchi, E. & Rogge, L. Chromatin remodeling by the SWI/SNF-like BAF complex and STAT4 activation synergistically induce IL-12Rbeta2 expression during human Th1 cell differentiation. EMB J 26, 1292-1302 (2007).
28. Stobie, L. et al. The role of antigen and IL-12 in sustaining Th1 memory cells in vivo: IL-12 is required to maintain memory/effector Th1 cells sufficient to mediate protection to an infectious parasite challenge. Proc Natl Acad Sci U S A 97, 8427-8432 (2000).
29. Park, A.Y., Hondowicz, B.D. & Scott, P. IL-12 is required to maintain a Th1 response during Leishmania major infection. J Immunol 165, 896-902 (2000).
30. Yap, G., Pesin, M. & Sher, A. Cutting edge: IL-12 is required for the maintenance of IFN-gamma production in T cells mediating chronic resistance to the intracellular pathogen, Toxoplasma gondii. J Immunol 165, 628-631 (2000).
31. Jacobson, N.G. et al. Interleukin 12 signaling in T helper type 1 (Th1) cells involves tyrosine phosphorylation of signal transducer and activator of transcription (Stat)3 and Stat4. J Exp Med 181, 1755-1762 (1995).
32. Winders, B.R., Schwartz, R.H. & Bruniquel, D. A distinct region of the murine IFN-gamma promoter is hypomethylated from early T cell development through mature naive and Th1 cell differentiation, but is hypermethylated in Th2 cells. J Immunol 173, 7377-7384 (2004).
33. Jones, B. & Chen, J. Inhibition of IFN-gamma transcription by site-specific methylation during T helper cell development. EMBO J 25, 2443-2452 (2006).
34. Li, E. Chromatin modification and epigenetic reprogramming in mammalian development. Nat Rev Genet 3, 662-673 (2002).
35. Chen, W.G. et al. Derepression of BDNF transcription involves calcium-dependent phosphorylation of MeCP2. Science 302, 885-889 (2003).
36. Martinowich, K. et al. DNA methylation-related chromatin remodeling in activity-dependent BDNF gene regulation. Science 302, 890-893 (2003).
37. Cedar, H. & Verdine, G.L. Gene expression. The amazing demethylase. Nature 397, 568-569 (1999).
38. Kim, M.S. et al. DNA demethylation in hormone-induced transcriptional derepression. Nature 461, 1007-1012 (2009).
39. Kress, C., Thomassin, H. & Grange, T. Active cytosine demethylation triggered by a nuclear receptor involves DNA strand breaks. Proc Natl Acad Sci U S A 103, 11112-11117 (2006).
40. Popp, C. et al. Genome-wide erasure of DNA methylation in mouse primordial germ cells is affected by AID deficiency. Nature 463, 1101-1105.
41. Metivier, R. et al. Cyclical DNA methylation of a transcriptionally active promoter. Nature 452, 45-50 (2008).
42. Kangaspeska, S. et al. Transient cyclical methylation of promoter DNA. Nature 452, 112-115(2008).
43. Eivazova, E.R. & Aune, T.M. Dynamic alterations in the conformation of the Ifng gene region during T helper cell differentiation. Proc Natl Acad Sci U S A 101, 251-256 (2004).
44. Wu, C.Y. et al. Distinct lineages of T(H)1 cells have differential capacities for memory cell generation in vivo. Nat Immunol 3, 852-858 (2002).
45. Tykocinski, L.O. et al. A critical control element for interleukin-4 memory expression in T helper lymphocytes. J Biol Chem 280, 28177-28185 (2005).
46. Morinobu, A., Kanno, Y. & O'Shea, J.J. Discrete roles for histone acetylation in human T helper 1 cell-specific gene expression. J Biol Chem 279, 40640-40646 (2004).
47. Wei, L. et al. Discrete roles of STAT4 and STAT6 transcription factors in tuning epigenetic modifications and transcription during T helper cell differentiation. Immunity 32, 840-851.
48. Kimmig, S. et al. Two subsets of naive T helper cells with distinct T cell receptor excision circle content in human adult peripheral blood. J Exp Med 195, 789-794 (2002).

## Claims

1. In vitro method for establishing functional cytokine memory for IFN-gamma in a cell and/or cell population via demethylation of one or more regulatory nucleic acid sequences associated with the cytokine-encoding nucleic acid sequence,
comprising the following steps:- provision of a cell population comprising CD4+ T-cells,
- subsequent activation of the cell population,
- isolation of an IFN-gamma expressing cell population from the activated cells, and
- application of IL-12 to the isolated IFN-gamma expressing cell population to induce DNA demethylation of one or more regulatory nucleic acid sequences associated with the IFN-gamma-encoding sequence.

2. Method according to any one of the preceding claims, **characterised in that** the IL-12 is applied to the cell population between 24 and 72 hours, preferably 48 hours, after initial activation and/or isolation of cytokine-expressing cells.

3. Method according to any one of the preceding claims, **characterised in that** the regulatory DNA sequences are promoter sequences, enhancer sequences, operator sequences or any other regulatory DNA element associated with and/or required for transcription of said cytokine encoding nucleic acid.

4. Method according to any one of the preceding claims, **characterised in that** the regulatory DNA sequences are the *IFNG* promoter, *IFNG* coding region and/or the *IFNG* CNS-1 sequence, preferably according to SEQ ID NO 1, SEQ ID NO 2, SEQ ID NO 3 and/or SEQ ID NO 4.

5. Method according to any one of the preceding claims, **characterised in that** demethylation of the regulatory DNA sequences occurs at CpG sequences, preferably CpG1: -4397; CpG2: -4375; CpG3: -4358; CpG4: -4323; CpG5: -4291; CpG6: -4276; CpG7: -4227 of SEQ ID NO 1, and/or CpG1: -495; CpG2: -372; CpG3: -295; CpG4: -186 of SEQ ID NO 2, and/or CpG1: -186; CpG2: -53; CpG3: +122; CpG4: +128; CpG5: +171 of SEQ ID NO 3, and/or CpG1: +122; CpG2: +128; CpG3: +171; CpG4: +296 of SEQ ID NO 4.

6. Memory T cell population that exhibits functional cytokine memory for IFN-gamma, produced by the method according to any one of claims 1 to 5.

7. Memory T cell population according to the preceding claim for use as a medicament in cell therapy, preferably in the treatment of cancer, autoimmune diseases and/or infectious diseases.

8. Method for identifying functional cytokine memory for IFN-gamma in a cell and/or cell population that comprises CD4+ T cells, preferably helper T cells (Th1) and/or Th1 cells that express IFN-gamma, via analysis of DNA methylation of one or more regulatory DNA sequences associated with a nucleic acid sequence encoding IFN-gamma.

9. Method for identifying functional cytokine memory for IFN-gamma according to the preceding claim, comprising analyzing DNA methylation of promoter sequences, enhancer sequences, operator sequences or any other regulatory DNA element associated with and/or required for transcription of IFN-gamma-encoding nucleic acid,

10. Method for identifying functional cytokine memory for IFN-gamma according to claim 8, comprising analyzing DNA methylation of the IFNG promoter, IFNG coding region and/or the IFNG-CNS-1 sequence according to SEQ ID NO 1, SEQ ID NO 2, SEQ ID NO 3 and/or SEQ ID NO 4.

11. Method for identifying functional cytokine memory for IFN-gamma according to claim 8, comprising analyzing DNA methylation of CpG sequences selected from CpG1: -4397; CpG2: -4375; CpG3: -4358; CpG4: -4323; CpG5: -4291; CpG6: -4276; CpG7: -4227 of SEQ ID NO 1, and/or CpG1: -495; CpG2: -372; CpG3: -295; CpG4: -186 of SEQ ID NO 2, and/or CpG1: -186; CpG2: -53; CpG3: +122; CpG4: +128; CpG5: +171 of SEQ ID NO 3, and/or CpG1: +122; CpG2: +128; CpG3: +171; CpG4: +296 of SEQ ID NO 4.

12. Method according to any one of claims 8 to 11, **characterised in that** low DNA methylation levels, in comparison to control cells represent the presence of cytokine memory, whereby the control cell is a naïve T cell, a CD4+ T cell and/or a CD8+ T cell that does not express the cytokine.

13. Method of disease diagnosis and/or monitoring disease progression, preferably before, during and/or after medical treatment, comprising
- providing a population of memory T cells comprising CD4+ T cells, preferably helper T cells (Th1) and/or Th1 cells that express IFN-gamma, followed by
- execution of the method for identifying functional cytokine memory according to any one of claims 8 to 11, whereby
- the proportion of memory T cells exhibiting functional cytokine memory for IFN-gamma provides a quantitative or semi-quantitative assessment of disease presence and/or progression.

14. Method of disease diagnosis and/or monitoring disease progression according to the previous claim, **characterised in that**
- the disease is Rheumatoid arthritis, and
- increased levels of DNA methylation of regulatory DNA elements, preferably those according to claims 9 to 11, in CD4+ memory T cells in comparison to healthy subjects indicates disease.

15. Method according to any one of claims 8 to 14, **characterised in that** the analysis of DNA methylation is carried out via bisulfite cloning and sequencing of genomic DNA, direct bisulfite sequencing, bisulfite-specific PCR sequencing, pyrosequencing, methylation-sensitive PCR, using preferably quantitative or real-time PCR, HPLC, mass spectrometry, microarray analysis, methylated DNA immunoprecipitation, methylated CpG amplification coupled to microarrays, or any other method for analyzing DNA methylation.

## Patentansprüche

1. Verfahren zur Etablierung eines funktionellen Zytokin-Gedächtnisses für IFN-gamma in einer Zelle oder Zellpopulation mittels Demethylierung einer oder mehreren regulatorischen Nukleinsäuresequenzen, die mit der Zytokin-kodierenden Nukleinsäuresequenzen assoziert sind, umfassend die folgenden Schritte:
- Bereitstellung einer Zellpopulation umfassend CD4+ T-Zellen,
- Anschließende Aktivierung der Zellpopulation,
- Isolierung einer IFN-gamma exprimierenden Zellpopulation aus den aktivierten Zellen, und
- Verabreichung von IL-12 zur isolierten IFN-gamma exprimierenden Zellpopulation, um DNA-Methylierung einer oder mehreren regulatorischen Nukleinsäuresequenzen, die mit der Zytokin-kodierenden Nukleinsäuresequenzen assoziert sind, zu induzieren.

2. Verfahren nach einem der vorherigen Ansprüche, **dadurch gekennzeichnet, dass** IL-12 zur Zellpopulation zwischen 24 und 72 Stunden, bevorzugt 48 Stunden, nach der initialen Aktivierung und/oder Isolierung der Zytokin-exprimierenden Zellen verabreicht wird.

3. Verfahren nach einem der vorherigen Ansprüche, **dadurch gekennzeichnet, dass** die regulatorische DNA Sequenzen Promoter-Sequenzen, Enhancer-Sequenzen oder Operator-Sequenzen sind, oder sonstige regulatorische DNA-Elemente sind, die mit der Transkription der Zytokin-kodierenden Nukleinsäure assoziert und/oder für die Transkription der Zytokin-kodierenden Nukleinsäure erforderlich sind.

4. Verfahren nach einem der vorherigen Ansprüche, **dadurch gekennzeichnet, dass** die regulatorische DNA Sequenzen der *IFNG*-Promoter, die *IFNG*-kodierende Region und/or die *IFNG* CNS-1-Sequenz sind, bevorzugt gemäß SEQ ID NO 1, SEQ ID NO 2, SEQ ID NO 3 und/oder SEQ ID NO 4.

5. Verfahren nach einem der vorherigen Ansprüche, **dadurch gekennzeichnet, dass** die Demethylierung der regulatorischen DNA Sequenzen an CpG-Sequenzen erfolgt, bevorzugt CpG1: -4397; CpG2: -4375; CpG3: -4358; CpG4: -4323; CpG5: -4291; CpG6: -4276; CpG7: -4227 gemäß SEQ ID NO 1, und/oder CpG1: -495; CpG2: -372; CpG3: -295; CpG4: -186 gemäß SEQ ID NO 2, und/oder CpG1: -186; CpG2: -53; CpG3: +122; CpG4: +128; CpG5: +171 gemäß SEQ ID NO 3, und/oder CpG1: +122; CpG2: +128; CpG3: +171; CpG4: +296 gemäß SEQ ID NO 4.

6. Gedächtnis-T-Zellpopulation, die ein funktionelles Zytokin-Gedächtnis für IFN-gamma aufweist, hergestellt nach dem Verfahren gemäß einem der Ansprüche 1 bis 5.

7. Gedächtnis-T-Zellpopulation nach dem vorherigen Anspruch zur Verwendung als Medikament in der Zelltherapie, bevorzugt in der Behandlung von Krebs, Autoimmunerkrankungen und/oder infektiösen Erkrankungen.

8. Verfahren zur Identifizierung eines funktionellen Zytokin-Gedächtnisses für IFN-gamma in einer Zelle oder Zellpopulation, die CD4+ T-Zellen umfasst, bevorzugt Helfer-T-Zellen (Th1) und/oder Th1-Zellen, welche IFN-gamma exprimieren, mittels Analyse der DNA-Methylierung einer oder mehreren regulatorischen DNA Sequenzen, die mit einer IFN-gamma kodierenden Nukleinsäuresequenz assoziiert sind.

9. Verfahren zur Identifizierung eines funktionellen Zytokin-Gedächtnisses für IFN-gamma nach dem vorherigen Anspruch, umfassend eine Analyse der DNA-Methylierung von Promoter-Sequenzen, Enhancer-Sequenzen, Operator-Sequenzen oder sonstige regulatorische DNA-Elemente, die mit der Transkription der IFN-gamma-kodierenden Nukleinsäure assoziert und/oder für die Transkription der IFN-gamma-kodierenden Nukleinsäure erforderlich sind.

10. Verfahren zur Identifizierung eines funktionellen Zytokin-Gedächtnisses für IFN-gamma nach Anspruch 8, umfassend eine Analyse der DNA-Methylierung des IFNG-Promoters, der IFNG-kodierende Region und/oder der IFNG-CNS-1-Sequenz gemäß SEQ ID NO 1, SEQ ID NO 2, SEQ ID NO 3 und/oder SEQ ID NO 4.

11. Verfahren zur Identifizierung eines funktionellen Zytokin-Gedächtnisses für IFN-gamma nach Anspruch 8, umfassend eine Analyse der DNA-Methylierung von CpG-Sequenzen ausgewählt aus der Gruppe umfassend: CpG1: -4397; CpG2: -4375; CpG3: -4358; CpG4: - 4323; CpG5: -4291; CpG6: -4276; CpG7: -4227 gemäß SEQ ID NO 1, und/oder CpG1: -495; CpG2: -372; CpG3: -295; CpG4: -186 gemäß SEQ ID NO 2, und/oder CpG1: -186; CpG2: -53; CpG3: +122; CpG4: +128; CpG5: +171 gemäß SEQ ID NO 3, und/oder CpG1: +122; CpG2: +128; CpG3: +171; CpG4: +296 gemäß SEQ ID NO 4.

12. Verfahren nach einem der Ansprüche 8 bis 11, **dadurch gekennzeichnet, dass** niedrige DNA-Methylierung, im Vergleich zu Kontrollzellen, die Anwesenheit von Zytokin-Gedächtnis darstellt, wobei die Kontrollzelle eine näive T-Zelle, eine CD4+ T-Zelle und/oder eine CD8+ T-Zelle ist, die das Zytokin nicht exprimiert.

13. Verfahren zur Diagnose einer Krankheit und/oder zur Überwachung des Krankheitsverlaufs, bevorzugt vor, während und/oder nach einer medizinischen Behandlung, umfassend
- Bereitstellung einer Zellpopulation umfassend CD4+ T-Zellen, bevorzugt Helfer-T-Zellen (Th1) und/oder Th1-Zellen, welche IFN-gamma exprimieren, gefolgt von
- Ausführung des Verfahrens zur Identifizierung von einem funktionellen Zytokin-Gedächtnis gemäß einem der Ansprüche 8 bis 11, wobei
- die Proportion der Gedächtnis-T-Zellen, die ein funktionelles Zytokin-Gedächtnis für IFN-gamma aufweisen, eine quantitative oder semi-quantitative Aussage zur Anwesenheit der Krankheit und/oder zum Krankheitsverlauf darstellt.

14. Verfahren zur Diagnose einer Krankheit und/oder zur Überwachung des Krankheitsverlaufs nach dem vorhergehenden Anspruch, **dadurch gekennzeichnet, dass**
- die Krankheit rheumatoide Arthritis ist, und
- erhöhte Mengen von DNA-Methylierung von regulatorischen DNA-Elemente, bevorzugt die gemäß den Ansprüchen 9 bis 11, in CD4+ Gedächtnis-T-Zellen im Vergleich zur gesunden Patienten auf die Anwesenheit der Krankheit hindeutet.

15. Verfahren nach einem der Ansprüche 8 bis 14, **dadurch gekennzeichnet, dass** die Analyse von DNA-Methylierung mittels Bisulfit-Klonierung und Sequenzierung genomische DNA, direkte Bisulfit-Sequenzierung, Bisulfit-spezifische PCR-Sequenzierung, Pyrosequenzierung, Methylierungssensible PCR, bevorzugt mittels quantitative oder echtzeit PCR, HPLC, Massenspektrometrie, Mikroarrayanalyse, methylierte DNA-Immunopräzipitation, methylierte CpG-Amplifizierung gekoppelt mit Mikroarrays, oder sonstige Verfahren zur Analyse von DNA-Methylierung erfolgt.

## Revendications

1. Procédé *in vitro* permettant d'établir une mémoire de cytokine fonctionnelle pour l'IFN gamma dans une cellule et/ou une population cellulaire par l'intermédiaire de la déméthylation d'une ou de plusieurs séquences d'acide nucléique régulatrices associées à la séquence d'acide nucléique codant pour la cytokine, comprenant les étapes suivantes :
- la fourniture d'une population cellulaire comprenant des lymphocytes T CD4+,
- l'activation subséquente de la population cellulaire,
- l'isolation d'une population cellulaire exprimant l'IFN gamma à partir des cellules activées, et
- l'application d'IL-12 à la population cellulaire exprimant l'IFN gamma isolée pour induire la déméthylation de l'ADN d'une ou de plusieurs séquences d'acide nucléique régulatrices associées à la séquence codant pour l'IFN gamma.

2. Procédé selon l'une quelconque des revendications précédentes, **caractérisé en ce que** l'IL-12 est appliquée à la population cellulaire entre 24 et 72 heures, de préférence 48 heures, après l'activation initiale et/ou l'isolation des cellules exprimant la cytokine.

3. Procédé selon l'une quelconque des revendications précédentes, **caractérisé en ce que** les séquences d'ADN régulatrices sont des séquences promoteurs, des séquences amplificateurs, des séquences opérateurs ou tout autre élément d'ADN régulateur associé et/ou nécessaire à la transcription dudit acide nucléique codant pour la cytokine.

4. Procédé selon l'une quelconque des revendications précédentes, **caractérisé en ce que** les séquences d'ADN régulatrices sont le promoteur de l'*IFNG,* la région codante de l'*IFNG* et/ou la séquence CNS-1 de l'*IFNG,* de préférence selon SÉQ ID NO : 1, SÉQ ID NO : 2, SÉQ ID NO : 3 et/ou SÉQ ID NO : 4.

5. Procédé selon l'une quelconque des revendications précédentes, **caractérisé en ce que** la déméthylation des séquences d'ADN régulatrices se produit au niveau des séquences de CpG, de préférence CpG1 : -4397 ; CpG2 : -4375 ; CpG3 : -4358 ; CpG4 : - 4323 ; CpG5 : -4291 ; CpG6 ; -4276 ; CpG7 : -4227 de SÉQ ID NO : 1, et/ou CpG -495 ; CpGs2 : -372 ; CpG3 : -295 ; CpG4 : -186 de SÉQ ID NO : 2, et/ou CpG1 : -186 ; CpGs2 : -53 ; CpG3 : +122 ; CpG4 : +128 ; CpG5 : +171 de SÉQ ID NO : 3, et/ou CpG1 : +122 ; CpG2 : +128 ; CpG3 : +171 ; CpG4 : +296 de SÉQ ID NO : 4.

6. Population de lymphocytes T mémoire qui présente une mémoire de cytokine fonctionnelle pour l'IFN gamma, produite par le procédé selon l'une quelconque des revendications 1 à 5.

7. Population de lymphocytes T mémoire selon la revendication précédente pour une utilisation en tant que médicament dans la thérapie cellulaire, de préférence dans le traitement du cancer, des maladies auto-immunes et/ou des maladies infectieuses.

8. Procédé d'identification d'une mémoire de cytokine fonctionnelle pour l'IFN gamma dans une cellule et/ou une population cellulaire qui comprend des lymphocytes T CD4+, de préférence des lymphocytes T auxiliaires (Th1) et/ou des cellules Th1 qui expriment l'IFN gamma, par l'intermédiaire de l'analyse de la méthylation de l'ADN d'une ou de plusieurs séquences d'ADN régulatrices associées à une séquence d'acide nucléique codant pour l'IFN gamma.

9. Procédé d'identification d'une mémoire de cytokine fonctionnelle pour l'IFN gamma selon la revendication précédente, comprenant l'analyse de la méthylation de l'ADN des séquences promoteurs, des séquences amplificateurs, des séquences opérateurs ou de tout autre élément d'ADN régulateur associé et/ou nécessaire à la transcription de l'acide nucléique codant pour l'IFN gamma.

10. Procédé d'identification d'une mémoire de cytokine fonctionnelle pour l'IFN gamma selon la revendication 8, comprenant l'analyse de la méthylation de l'ADN du promoteur de l'IFNG, de la région codante de l'IFNG et/ou de la séquence CNS-1 de l'IFNG selon SÉQ ID NO : 1, SÉQ ID NO : 2, SÉQ ID NO : 3 et/ou SÉQ ID NO : 4.

11. Procédé d'identification d'une mémoire de cytokine fonctionnelle pour l'IFN gamma selon la revendication 8, comprenant l'analyse de la méthylation de l'ADN de séquences de CpG choisies parmi CpG1 : -4397 ; CpG2 : -4375 ; CpG3 : -4358 ; CpG4 : -4323 ; CpG5 : -4291 ; CpG6 ; -4276 ; CpG7 : -4227 de SÉQ ID NO : 1, et/ou CpG1 : -495 ; CpGs2 : -372 ; CpG3 : -295 ; CpG4 : -186 de SÉQ ID NO : 2, et/ou CpG1 : -186 ; CpGs2 : -53 ; CpG3 : +122 ; CpG4 : +128 ; CpG5 : +171 de SÉQ ID NO : 3, et/ou CpG1 : +122 ; CpG2 : +128 ; CpG3 : +171 ; CpG4 : +296 de SÉQ ID NO : 4.

12. Procédé selon l'une quelconque des revendications 8 à 11, **caractérisé en ce que**
des taux bas de méthylation de l'ADN, comparativement à des cellules témoins représentent la présence d'une mémoire de cytokine, moyennant quoi la cellule témoin est un lymphocyte T naïf, un lymphocyte T CD4+ et/ou un lymphocyte T CD8+ qui n'exprime pas la cytokine.

13. Procédé de diagnostic d'une maladie et/ou de suivi de la progression d'une maladie, de préférence avant, durant et/ou après un traitement médical, comprenant
- la fourniture d'une population de lymphocytes T mémoire comprenant des lymphocytes T CD4+, de préférence des lymphocytes T auxiliaires (Th1) et/ou des cellules Th1 qui expriment l'IFN gamma, suivie de
- l'exécution du procédé d'identification d'une mémoire de cytokine fonctionnelle selon l'une quelconque des revendications 8 à 11, moyennant quoi
- la proportion de lymphocytes T mémoire présentant une mémoire de cytokine fonctionnelle pour l'IFN gamma fournit une estimation quantitative ou semi-quantitative de la présence et/ou de la progression d'une maladie.

14. Procédé de diagnostic d'une maladie et/ou de suivi de la progression d'une maladie selon la revendication précédente, **caractérisé en ce que**
- la maladie est la polyarthrite rhumatoïde, et
- des taux accrus de méthylation d'ADN des éléments d'ADN régulateurs, de préférence ceux selon les revendications 9 à 11, dans les lymphocytes T mémoire CD4+ comparativement à des sujets en bonne santé indiquent une maladie.

15. Procédé selon l'une quelconque des revendications 8 à 14, **caractérisé en ce que**
l'analyse de la méthylation de l'ADN est réalisée par l'intermédiaire du clonage et du séquençage au bisulfite de l'ADN génomique, du séquençage direct au bisulfite, du séquençage par PCR spécifique du bisulfite, du pyroséquençage, de la PCR sensible à la méthylation, en utilisant de préférence une PCR quantitative ou en temps réel, de la CLHP, de la spectrométrie de masse, de l'analyse de micropuces, de l'immunoprécipitation de l'ADN méthylé, de l'amplification des CpG méthylés couplés à des micropuces, ou de tout autre procédé pour l'analyse de la méthylation de l'ADN.
